# EUROPEAN PATENT APPLICATION

(11) **EP 3 533 460 A1**
(43) Date of publication of application: **04.09.2019**
(21) Application number: 18305229.9
(22) Date of filing: 02.03.2018
(51) Int. Cl.: A61K 39/00, C07K 16/40

(54) **THERAPEUTIC ANTI-SPLA2-GIB ANTIBODIES AND THE USES THEREOF**

(71) Applicant: Diaccurate, 75008 Paris (FR)
(72) Inventor: TAMARIT, Blanche, 75014 Paris (FR); THÈZE, Jacques, 75007 Paris (FR)
(74) Representative: Cabinet Becker et Associés

(57) **Abstract**

The present invention relates to human or humanized antibodies which bind PLA2-GIB, their production and the uses thereof. These antibodies exhibit advantageous characteristics, in particular for therapeutic and diagnostic purposes.

## Description

The present invention relates to antibodies which bind PLA2-GIB, their production and the uses thereof. These antibodies exhibit advantageous characteristics, in particular for therapeutic and diagnostic purposes.

### BACKGROUND OF THE INVENTION

The group IB pancreatic secreted phospholipase A2 (PLA2-GIB) is a low molecular weight (14 kD), highly stable (7 disulfide bonds), secreted protein, that catalyzes the hydrolysis of the sn-2 fatty acyl bond of phospholipids to release free fatty acids and lysophospholipids (Lambeau & Gelb 2008). In human, the *PLA2-G1B* gene is mainly expressed in the pancreas, with weaker expression levels in the duodenum, jejunum, and stomach (Eskola et al. 1983a) (Nevalainen & Haapanen 1993). Marginal expression has been described in other tissues, such as the lung, eyes (Kolko et al. 2007) and testis. In human pancreas, PLA2-GIB is mostly synthesized in apical zymogen granule portion of pancreatic acinar cells (Eskola et al. 1983a). sPLA2-GIB has also been detected in insulin secretory granules of pancreatic islet ß-cells and to be co-secreted with insulin from glucose-stimulated islets (Ramanadham et al. 1998). The enzyme is released in the pancreatic juice as an inactive pro-form and is activated by proteolytic cleavage of a pro-peptide to generate the mature secreted form (sPLA2-GIB). In vitro experiments suggested that trypsin and plasmin are able to activate pro-sPLA2-GIB with good potency (Nakano et al. 1994).

In addition to a role in the gastrointestinal tract, PLA2-GIB is also circulating in human plasma ((Nishijima et al. 1983) (Eskola et al. 1983b) (Sternby 1984) (Nevalainen et al. 1985) (Kitagawa et al. 1991)). Recently, PLA2-GIB was identified as playing a crucial role in the mechanism underlying the unresponsiveness of CD4 T lymphocytes in viremic HIV-infected patients. In particular, it was demonstrated that sPLA2-IB is the protein responsible for the chronic inactivation of CD4 T lymphocytes, which drives them into an aberrant state of activation differentiation which renders them refractory to certain physiological signals such as those delivered by interleukin-7 (THEZE Jacques et al. 2015).

There is therfore a need for antibodies against PLA2-GIB that allow an inhibition of the enzymatic activity of PLA2-GIB.

### SUMMARY OF THE INVENTION

The present invention provides anti-PLA2-GIB antibodies and methods of using the same. More particularly, the invention provides therapeutic antibodies against human sPLA2-GIB that can neutralize an enzymatic activity of sPLA2-GIB.

An object of the invention more particularly relates to human or humanized antibodies, or derivatives thereof, that bind human sPLA2-GIB.

Preferred human or humanized antibodies of the invention bind an epitope comprising at least one amino acid residue selected from W3, R6, K7, K10, C77, Y75, G79 and S80 of human sPLA2-GIB. Further preferred antibodies of the invention are neutralizing antibodies. Specific examples of such antibodies are antibodies #2B, #2B1 and #2B2.

Another object of the invention relates to a nucleic acid molecule encoding an antibody or derivative as defined above, or a light or heavy chain thereof, or a variable domain thereof.

The invention also relates to a vector comprising a nucleic acid as defined above, as well as to a recombinant host cell containing such a vector or nucleic acid molecule.

A further object of the invention is a pharmaceutical composition comprising (i) an antibody or derivative, a nucleic acid, a vector, or a recombinant host cell, as defined above, and (ii) a pharmaceutically acceptable carrier or excipient.

The invention also relates to an antibody or derivative, a nucleic acid, a vector, a recombinant host cell, or a composition, as defined above, for use as a medicament, particularly in treatment of an immune disorder, in a subject in need thereof.

The invention also relates to a method for treating a subject in need thereof, particularly a subject having an immune disorder, comprising administering to the subject an antibody or derivative, a nucleic acid, a vector, a recombinant host cell, or a composition, as defined above.

A further object of the invention relates to a method for producing an antibody as defined above, comprising culturing a cell as defined above under conditions suitable for expression of the antibody, and optionally recovering said antibody.

The therapeutic antibodies of the invention may be used in any mammal, particularly any human subject in need thereof. They are suitable to inhibit PLA2-GIB and correct any disease associated with undesirable activity of said protein.

### LEGEND TO THE DRAWINGS

**Figure 1****:** Properties of monoclonal antibody 14G9. A: inhibition of sPLA2-GIB enzymatic activity. B: dilution curves in indirect ELISA. C: dilution curves in competitive ELISA.
**Figure 2****:** Inhibition monoclonal antibody 14G9 of the inhibitory effect of plasma isolated from HIV-1 infected patients on the nuclear translocation of phosphoSTAT5 in CD4 T cells from one healthy donor. No effect was observed using an isotype control mAb.
**Figure 3****:** Inhibition by 10 µg of monoclonal antibody 14G9 of the inhibitory effect of plasma isolated from HIV-1 infected patients on the nuclear translocation of phosphoSTAT5 in CD4 T cells from 5 different healthy donors. No effect was observed using 10 µg of an isotype control mAb or the non-inhibitory #6F7 mAb.
**Figure 4****:** *In vivo* inhibition by 1 mg of monoclonal antibody 14G9 of the inhibitory effect of 100 µg of sPLA2-GIB on the nuclear translocation of phosphoSTAT5 in CD4 T cells isolated from mice splenocytes. No significant effect was observed when injecting 900 µg of an isotype control mAb.
**Figure 5****:** Estimation of affinity of 14G9 humanized variants by competitive ELISA
**Figure 6****:** Normalized thermal denaturation of humanized antibodies #1A, #1B, #2A and #2B.
**Figure 7****:** SEC Analysis of #2B. Upper panel, SEC chromatogram of #2B. Middle panel, zoomed baseline. Lowe panel, zoomed baseline with peak assignment.
**Figure 8****:** Normalized thermal denaturation of humanized antibodies #2B1 and #2B2.
**Figure 9****:** Flow cytometry analysis of the monomeric IgG binding of humanized antibodies #2B, #2B1 and #2B2 to human FcyRs.
**Figure 10****:** Flow cytometry analysis of the immune complexes binding of #2B to human FcyRs.
**Figure 11****:** Flow cytometry analysis of residual binding of antigen on antibody-sensitized cells of humanized antibodies #2B, #2B1 and #2B2 to human FcyRs.
**Figure 12****:** Crystal structure of the Fab#2B2/sPLA2-IB complex. sPLA2-GIB backbones are depicted in grey and purple, #2B2 Fab light chain is in light blue and pink and #2B2 Fab heavy chain is in green and yellow (**A**). Non-covalent bonds in Fab#2B2/sPLA2-GIB complex, sPLA2-GIB backbone is in yellow, #2B2 Fab light chain is in light blue and #2B2 Fab heavy chain is in green (**B**).
**Figure 13****:** Inhibition by 14G9 or #2B of the inhibitory effect of sPLA2-GIB (**A**) or of plasma isolated from HIV-1 infected patients (**B**) on the nuclear translocation of phosphoSTAT5 in CD4 T cells from one healthy donor.
**Figure 14****:** *In vivo* inhibition by 1 or 2 mg of #2B2 of the inhibitory effect of 100 µg of sPLA2-GIB on the nuclear translocation of phosphoSTAT5 in CD4 T cells isolated from mice splenocytes. No significant effect was observed when injecting 1 mg µg of an isotype control mAb.
**Table 1:** Percentage identity between mouse V genes or their humanized versions with variable human germline used as acceptor sequences.
**Table 2:** Details of the differences in sequence across the V genes between the humanized versions and their corresponding human germlines.
**Table 3:** Inhibition of sPLA2-GIB enzymatic activity by humanized antibodies. Results are expressed as % of control.
**Table 4:** Estimated IC50 of the different humanized antibodies obtained by competitive ELISA towards recombinant human sPLA2-GIB and recombinant human pro-sPLA2-GIB.
**Table 5:** Differential Scanning Calorimetry of humanized antibodies. Tm are denaturation/melting temperatures. T onset are the temperatures at which the unfolding transition begins. T1/2 are the width of the transition at half height of the peak. ΔH are the calorimetric enthalpy of unfolding and reflect the disruption of intramolecular interactions of the protein. Total Area are the entire enthalpy of unfolding (total area under the thermogram) and reflect thermodynamic stability.
**Table 6:** SEC-HPLC analysis of #1A, #1B, #2A and #2B humanized antibodies.
**Table 7:** Determination of Kd towards recombinant human sPLA2-GIB and recombinant human pro-sPLA2-GIB by Surface Plasmon Resonance of the #2B, #2B1 and #2B2 humanized antibodies.
**Table 8:** Differential Scanning Calorimetry of #2B1 and #2B2 humanized antibodies. Tm are denaturation/melting temperatures. T onset are the temperatures at which the unfolding transition begins. T1/2 are the width of the transition at half height of the peak. ΔH are the calorimetric enthalpy of unfolding and reflect the disruption of intramolecular interactions of the protein. Total Area are the entire enthalpy of unfolding (total area under the thermogram) and reflect thermodynamic stability.
**Table 9:** SEC-HPLC analysis of the #2B1 and #2B2 humanized antibodies.
**Table 10:** Imaged capillary isoelectric focusing (icIEF) analysis of the #2B, #2B1 and #2B2 humanized antibodies. Average apparent pI of the different charge variants present in the samples.

### DETAILED DESCRIPTION OF THE INVENTION

### Antibodies

The present invention is based on the generation of novel human and humanized antibodies that are therapeutically useful to effectively and selectively modulate the activity of PLA2-GIB in a human subject.

As used herein, the term "PLA2-GIB" designates group IB pancreatic phospholipase A2. PLA2-GIB has been identified and cloned from various mammalian species. The human PLA2-GIB protein is disclosed, for instance, in Lambeau and Gelb (2008). The sequence is available on Genbank No. NP_000919.

The amino acid sequence of an exemplary human PLA2-GIB is shown below (SEQ ID NO: 1).

MKLLVLAVLL TVAAADSGIS PRAVWQFRKM IKCVIPGSDP
FLEYNNYGCY CGLGGSGTPV DELDKCCQTH DNCYDQAKKL DSCKFLLDNP
YTHTYSYSCS GSAITCSSKN KECEAFICNC DRNAAICFSK APYNKAHKNL
DTKKYCQS

Amino acids 1 to 15 of SEQ ID NO: 1 (underlined) are a signal sequence, and amino acids 16 to 22 of SEQ ID NO: 1 (in bold) are a propeptide sequence.

Within the context of the invention, the term "PLA2-GIB" designates preferably human PLA2-GIB.

The human PLA2-GIB protein may be present under two distinct forms: an inactive pro form (pro-sPLA2-GIB), which is activated by proteolytic cleavage of a pro-peptide, leading to the mature secreted form (sPLA2-GIB). The term PLA2-GIB includes any form of the protein, such as the pro-form and/or the mature form. Typically, the mature secreted form comprises the sequence of amino acid residues 23-148 of SEQ ID NO: 1 (SEQ ID NO: 14) or any natural variants thereof, such as variants resulting from polymorphism or splicing.

### SEQ ID NO: 14:

AVWQFRKMIK CVIPGSDPFL EYNNYGCYCG LGGSGTPVDE
LDKCCQTHDN CYDQAKKLDS CKFLLDNPYT HTYSYSCSGS AITCSSKNKE
CEAFICNCDR NAAICFSKAP YNKAHKNLDT KKYCQS

The term "pro-sPLA2-GIB" or "proPLA2-GIB" designates preferably a protein comprising the sequence of amino acid residues 16-148 of SEQ ID NO: 1 or any natural variants thereof, such as variants resulting from polymorphism or splicing.

Applicant has generated several monoclonal antibodies against sPLA2-GIB. They have found that one of them, antibody #14G9, exhibits remarkable neutralizing activity and is selective for sPLA2-GIB. Applicant further characterized this monoclonal antibody, its variable regions as well as the epitope thereof. In particular, as shown in the experimental section, antibody 14G9 binds an epitope comprising amino acid residues W3, R6, K7, K10, C77, Y75, G79 and S80 of human sPLA2-GIB, by reference to SEQ ID NO: 14. Applicant was also able to produce humanized antibodies based on 14G9, and to demonstrate their remarkable activity in vitro and in vivo.

The invention thus relates to isolated human or humanized antibodies against PLA2-GIB.

The invention more specifically provides human or humanized antibodies that selectively bind to and neutralize human PLA2-GIB.

"Humanized" forms of non-human (e.g., murine) antibodies are chimeric antibodies which contain minimal sequences derived from non-human immunoglobulin grafted into a human scaffold (e.g., human constant domain of antibody chains). For the most part, humanized antibodies are human immunoglobulins (recipient antibody) in which hypervariable region residues of the recipient are replaced by hypervariable region residues from a non-human species (donor antibody) such as mouse, rat, rabbit or nonhuman primate having the desired specificity, affinity, and capacity. Furthermore, humanized antibodies may comprise residues which are not found in the recipient antibody or in the donor antibody. These modifications are made to further refine antibody performance. In general, the humanized antibody comprises substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also comprises at least a portion of an immunoglobulin constant region (Fc), typically that of a human immunoglobulin (Jones et al., Nature 321:522-525 (1986); Reichmann et al., Nature 332:323.329 (1988); and Presta, Curr. Op. Struct. Biol. 2:593-596 (1992)).

A "human antibody" is an antibody having an amino acid sequence which corresponds to that of an antibody produced by a human and/or which has been made using any of the techniques for making human antibodies as disclosed herein. Human antibodies can be produced using various techniques known per se in the art, including phage-display libraries (Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE™ technology) and replaced by human loci. See also, for example, Li et al., Proc. Natl. Acad. Sci. USA, 103:3557-3562 (2006) regarding human antibodies generated via a human B-cell hybridoma technology.

Antibodies of the invention may be polyclonals, monoclonals, or derivatives thereof that retain the same antigenic specificity. The antibodies of the invention are preferably monoclonal antibodies, i.e., antibodies that are each directed against a single antigenic site. Monoclonal antibodies of the present invention may be prepared by methods known per se in the art, such as by the hybridoma method first described by Kohler et al., Nature 256:495 (1975), or by recombinant DNA methods. They may also be isolated from phage antibody libraries using techniques described in Clackson et al., Nature 352:624-628 (1991) and Marks et al., J. Mol. Biol. 222:581-597 (1991), for example.

The invention particularly provides human or humanized monoclonal antibodies which selectively bind to and neutralize human PLA2-GIB.

In preferred embodiments, the antibodies of the invention are humanized monoclonal antibodies.

In a more preferred embodiment, the invention relates to human or humanized monoclonal antibodies that bind human sPLA2-GIB, preferably an epitope comprising at least one amino acid residue selected from W3, R6, K7, K10, C77, Y75, G79 and S80 of human sPLA2-GIB (by reference to SEQ ID NO: 14), more preferably an epitope comprising at least 2 or at least 3 amino acid residues selected from W3, R6, K7, K10, C77, Y75, G79 and S80 of human sPLA2-GIB, further more preferably an epitope comprising at least 4, at least 5, at least 6 or at least 7 amino acid residues selected from W3, R6, K7, K10, C77, Y75, G79 and S80 of human sPLA2-GIB. The invention shows antibodies that bind such residues exhibit potent neutralizing activity and represent valuable therapeutic agents for in vivo inhibition of human PLA2-GIB.

In a particular embodiment, the antibodies or derivatives of the invention competitively inhibit the binding of monoclonal antibody 14G9 to human sPLA2-GIB, said monoclonal antibody 14G9 comprising a light chain variable region comprising SEQ ID NO: 2 and a heavy chain variable region comprising SEQ ID NO: 3.

In another particular embodiment, the antibodies or derivatives of the invention competitively inhibit the binding of monoclonal antibody #2B, to human sPLA2-GIB, said monoclonal antibody #2B comprising a light chain comprising or consisting of SEQ ID NO: 4 and a heavy chain comprising or consisting of SEQ ID NO: 5.

In a further particular embodiment, the antibodies or derivatives of the invention competitively inhibit the binding of monoclonal antibody #2B1, to human sPLA2-GIB, said monoclonal antibody #2B1 comprising a light chain comprising or consisting of SEQ ID NO: 4 and a heavy chain comprising or consisting of SEQ ID NO: 6.

In another particular embodiment, the antibodies or derivatives of the invention competitively inhibit the binding of monoclonal antibody #2B2, to human sPLA2-GIB, said monoclonal antibody #2B2 comprising a light chain comprising or consisting of SEQ ID NO: 4 and a heavy chain comprising or consisting of SEQ ID NO: 7.

The term "competitively inhibits" indicates that the antibody can reduce or inhibit or displace the binding of a reference antibody to sPLA2-GIB. Competition assays can be performed using standard techniques such as, for instance, competitive ELISA or other binding assays. Typically, a competitive binding assay involves a purified target antigen, generally bound either to a solid substrate or cells, an unlabeled test antibody and a labeled reference antibody. Competitive inhibition is measured by determining the amount of labeled antibody bound in the presence of the test antibody. Usually the test antibody is present in excess, such as about 5 to 500 times the amount of reference antibody. Typically, for ELISA, the test antibody is in 100X excess, and for enzymatic methods, the test antibody in in 10X excess. When a test antibody present in excess inhibits or displaces at least 70% of the binding of the reference antibody to the antigen, it is considered as competitively inhibiting said reference antibody. In a specific embodiment, when a test antibody present in 100X excess inhibits or displaces at least 70%, more preferably at least 80% of the binding of the reference antibody to the antigen in ELISA, it is considered as competitively inhibiting said reference antibody. Preferred competing antibodies bind epitopes that share common amino acid residues.

In a particular embodiment, the invention relates to a monoclonal antibody comprising:
(i) a light chain variable region comprising a CDR-L1, a CDR-L2, a CDR-L3 and a FR-L, wherein the CDR-L1, CDR-L2 and/or CDR-L3 consists, or consists essentially, of the CDR-L1, CDR-L2 and CDR-L3, respectively, of the light chain variable region of SED ID NO: 2, and wherein a FR-L is of a human immunoglobulin sequence; and
(ii) a heavy chain variable region comprising a CDR-H1, a CDR-H2, a CDR-H3 and a FR-H, wherein the CDR-H1, CDR-H2 and/or CDR-H3 consists, or consists essentially, of the CDR-H1, CDR-H2 and CDR-H3, respectively, of the heavy chain variable region of SEQ ID NO: 3, and wherein a FR-H is of a human immunoglobulin sequence.

The "variable region" of an antibody refers to the amino-terminal domains of the heavy or light chain ("VH" or "VL"), which contain the antigen-binding sites. A light or heavy chain variable region (VL or VH) generally consists of a framework region ("FR") interrupted by three hypervariable regions referred to as "complementarity determining regions" or "CDRs". The extent of the framework region and CDRs have been precisely defined, for example as in Kabat (see "Sequences of Proteins of Immunological Interest," E. Kabat et al., U.S. Department of Health and Human Services, (1983)), and as in Chothia.

With reference to SEQ ID NO: 2, the three CDR regions correspond to the following amino acid residues:
- CDR-L1: amino acid residues QDVSTA,
- CDR-L2: amino acid residues WAS,
- CDR-L3: amino acid residues QQDYSTPPT.

With reference to SEQ ID NO: 3, the three CDR regions correspond to the following amino acid residues:
- CDR-H1: amino acid residues GYTFTNYW,
- CDR-H2: amino acid residues IDPSDTRT,
- CDR-H3: amino acid residues ARQTLYYEALDY.

In a particular embodiment, the invention relates to a monoclonal antibody selected from:
- a monoclonal antibody comprising a light chain comprising, consisting essentially of, or consisting of SEQ ID NO: 4 and a heavy chain comprising, consisting essentially of, or consisting of SEQ ID NO: 5 (clone #2B);
- a monoclonal antibody comprising a light chain comprising, consisting essentially of, or consisting of SEQ ID NO: 4 and a heavy chain comprising, consisting essentially of, or consisting of SEQ ID NO: 6 (clone #2B1);
- a monoclonal antibody comprising a light chain comprising, consisting essentially of, or consisting of SEQ ID NO: 4 and a heavy chain comprising, consisting essentially of, or consisting of SEQ ID NO: 7 (clone #2B2); and
- derivatives thereof.

The term "antibody derivative", as used herein, refers to an antibody which retains the antigenic specificity of a reference antibody but wherein one or more amino acid residues are (chemically, or biologically) modified to improve its properties.

Examples of such chemical modifications include, e.g. by alkylation, PEGylation, acylation, ester or amide formation, and the like. In particular, a derivative is an antibody as disclosed herein that is modified to contain one or more additional non-proteinaceous moieties such as water soluble polymers. Examples of water soluble polymers include, but are not limited to, PEG, copolymers of ethylene glycol/propylene glycol, carboxymethylcellulose, dextran and polyvinyl alcohol.

Derivatives may also be generated to increase or decrease the extent to which the antibody is glycosylated. Addition or deletion of glycosylation sites to an antibody may be conveniently accomplished by altering the amino acid sequence such that one or more glycosylation sites is created or removed. Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region (see e.g., Wright et al. TIBTECH, 1997, 15:26-32). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%). The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Examples of publications related to "defucosylated" or "fucose-deficient" antibody variants include, but are not limited to, Okazaki et al. J. Mol. Biol. 336: 1239-1249 (2004) and Yamane-Ohnuki N, Satoh M. mAbs. 2009;1:230-236. Examples of cell lines capable of producing defucosylated antibodies include Led 3 CHO cells deficient in protein fucosylation (Ripka et al. Arch. Biochem. Biophys. 249:533-545 (1986)), and knockout cell lines, such as alpha- 1 ,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006)).

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

The term derivative also includes immunoconjugates comprising an anti-sPLA2-GIB antibody as defined above conjugated to one or more heterologous molecule(s), including but not limited to a cytotoxic agent, a detectable moiety such as a fluorescent moiety, a diagnostic radioisotope or an imaging agent; or to a solid support, such as agarose beads or the like. Examples of cytotoxic agents include, but are not limited to chemotherapeutic agents or drugs, growth inhibitory agents, toxins (e.g., protein toxins, enzymatically active toxins of bacterial, fungal, plant, or animal origin, or fragments thereof), or radioactive isotopes. Conjugates of an antibody and cytotoxic agent may be made using a variety of bifunctional protein coupling agents well known by the skilled person. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52: 127-131 (1992)) may be used.

The antibodies of the invention are typically "isolated", e.g., have been separated from at least one component of their natural environment. In particular, the antibodies may be purified to greater e.g., at least 95%, at least 96%; at least 97%, at least 98% or at least 99% purity as determined by, for example, electrophoretic (e.g., SDS-PAGE, isoelectric focusing (IEF), capillary electrophoresis) or chromatographic (e.g., ion exchange or reverse phase HPLC) techniques. For review of methods for assessment of antibody purity, see, e.g. Flatman et al., J. Chromatogr. B 848:79-87 (2007).

As discussed above, the antibodies of the invention are essentially neutralizing antibodies, i.e., they are able to at least partially inhibit an activity of PLA2-GIB.

sPLA2-GIB catalyzes the hydrolysis of the sn-2 fatty acyl bond of phospholipids to release free fatty acids and lysophospholipids. Particular antibodies of the invention inhibit an enzymatic activity of sPLA2-GIB, such as the hydrolysis of the sn-2 fatty acyl bond of phospholipids. Methods for testing such a property are disclosed in detail in the experimental section.

Further particular antibodies of the invention inhibit binding of sPLA2-GIB to a substrate thereof.

Further particular antibodies of the invention inhibit sPLA2-GIB-mediated inhibition of IL-7-induced phospho-STAT5 nuclear translocation in CD4 T cells. Methods for testing such a property are disclosed in detail in the experimental section.

The neutralizing activity of the antibody or derivative can be determined in vitro or in vivo using e.g., binding or biological assays, such as tests as described in the experimental section. Inhibition/neutralization may be complete or partial. In particular, the antibodies may inhibit 10% or more of the tested activity, preferably 20% or more, 30% or more, 40% or more, 50% or more.

In preferred embodiments, the antibodies are IgG, e.g., gG1, IgG2, IgG3 or IgG4.

Preferred antibodies selectively bind sPLA2-GIB, i.e., exhibit preferential binding to sPLA2-GIB as compared to other molecules. Selectivity can be determined by competitive ELISA or Biacore assays. The difference in affinity/avidity that marks selectivity can be any detectable preference, such as e.g., a ratio of more than 1:1.1, or more than about 1:5, 1:10, 1:100, 1:1000 or more.

The antibodies or derivatives of the invention may be isolated and preserved using conventional methods and media. They may be lyophilized. They may also be frozen.

### Nucleic acids, vectors, host cells and recombinant production

In another aspect, the present invention concerns nucleic acid molecules encoding an antibody of the invention, or a light or heavy chain thereof, or a variable domain thereof, or a nucleic acid complementary to said encoding sequence. Preferably, the nucleic acid is an isolated or purified nucleic acid.

The nucleic acid can be DNA (cDNA or gDNA), RNA, or a mixture thereof. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis. The nucleic acid according to the invention may be deduced from the sequence of the antibody according to the invention and codon usage may be adapted according to the host cell in which the nucleic acid shall be transcribed. These steps may be carried out according to methods well known to one of skill in the art and some of which are described in the reference manual Sambrook *et al.* (Sambrook J, Russell D (2001) Molecular cloning: a laboratory manual, Third Edition Cold Spring Harbor).

The nucleic acid of the invention may encode an amino acid sequence comprising the light chain and/or an amino acid sequence comprising the heavy chain of the antibody, or may be complementary to such encoding sequence.
specific examples of such nucleic acid sequences include the sequences provided as SEQ ID NOs: 10-13.

The present invention further provides a vector comprising a nucleic acid of the invention. Optionally, the vector may comprise several nucleic acids of the invention. In particular, the vector may comprise a nucleic acid of the invention operably linked to a regulatory region, i.e. a region comprising one or more control sequences. Optionally, the vector may comprise several nucleic acids of the invention operably linked to several regulatory regions.

The term "control sequences" means nucleic acid sequences necessary for expression of a coding region. Control sequences may be endogenous or heterologous. Well-known control sequences and currently used by the person skilled in the art will be preferred. Such control sequences include, but are not limited to, promoter, signal peptide sequence and transcription terminator.

The term "operably linked" means a configuration in which a control sequence is placed at an appropriate position relative to a coding sequence, in such a way that the control sequence directs expression of the coding region.

The present invention further relates to the use of a nucleic acid or vector according to the invention to transform, transfect or transduce a host cell.

The present invention also provides a host cell comprising one or several nucleic acids of the invention and/or one or several vectors of the invention.

The term "host cell" also encompasses any progeny of a parent host cell that is not identical to the parent host cell due to mutations that occur during replication.

Suitable host cells for cloning or expression of antibody-encoding vectors include prokaryotic or eukaryotic cells described herein.

For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell lysate in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22: 1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See, e.g., US Patent Nos. 5,959,177, 6,040,498, 6,420,548, 7,125,978, and 6,417,429.

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse Sertoli cells (TM4 cells as described, e.g., in Mather, Biol. Reprod. 23:243-251 (1980)); monkey kidney cells (CV1); African green monkey kidney cells (VERO- 76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N. Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255- 268 (2003).

The present invention also concerns a method for producing an antibody of the invention, comprising culturing a host cell comprising a nucleic acid of the invention or a vector of the invention, as provided above, under conditions suitable for expression of the antibody, and optionally recovering the antibody from the host cell or from the host cell culture medium. Optionally, the recovered antibody may be further purified or isolated. Suitable media, culture conditions and production method are well-known by the skilled person and can be easily chosen according to the host cell and the antibody to be produced.

### Pharmaceutical compositions

The present invention further relates to pharmaceutical compositions comprising an antibody, a nucleic acid, a vector or a host cell of the invention. The composition may comprise one or several antibodies of the invention, one or several nucleic acid of the invention and/or one or several vectors of the invention and/or one or several host cells of the invention. Preferably, the pharmaceutical composition comprises one or several antibodies of the invention.

Pharmaceutical compositions comprising an antibody of the invention can be formulated according to known methods to prepare pharmaceutically useful compositions, whereby the antibody having the desired degree of purity is mixed with optional physiologically acceptable carriers, excipients or stabilizers (Remington: The Science and Practice of Pharmacy 20th edition (2000)), in the form of aqueous solutions, lyophilized or other dried formulations.

As used herein, the term "pharmaceutical formulation" or "pharmaceutical composition" refers to a preparation which is in such form as to permit the biological activity of the active ingredient to be effective, and which contains no additional components which are unacceptably toxic to a subject to which the formulation would be administered. Preferably, such formulations are sterile, i.e. aseptic or free from all living microorganisms and their spores.

Acceptable carriers, excipients, or stabilizers are nontoxic to recipients at the dosages and concentrations employed, and include, but are not limited to, buffering agents, stabilizing agents, preservatives, isotonifiers, non-ionic detergents, antioxidants and other miscellaneous additives.

Buffering agents help to maintain the pH in the range which approximates physiological conditions. They are preferably present at concentration ranging from about 1 mM to about 50 mM. Suitable buffering agents for use with the present invention include, but are not limited to, both organic and inorganic acids and salts thereof such as citrate, succinate, tartrate, fumarate, gluconate, oxalate, lactate and acetate buffers, as well as phosphate buffers, histidine buffers and trimethylamine salts such as Tris.

Preservatives may be added to retard microbial growth, and may be added in amounts ranging from 0.2%-1% (w/v). Suitable preservatives for use with the present invention include, but are not limited to, phenol, butyl or benzyl alcohol; meta-cresol; alkyl parabens such as methyl or propyl paraben; octadecyldimethylbenzyl ammonium chloride, benzalkonium halides (e.g., chloride, bromide, iodide); hexamethonium or benzethonium chloride; catechol; resorcinol; cyclohexanol; and 3-pentanol.

Isotonifiers may be added to ensure isotonicity of liquid compositions of the present invention and include polhydric sugar alcohols, preferably trihydric or higher sugar alcohols, such as glycerin, erythritol, arabitol, xylitol, sorbitol and mannitol.

Stabilizing agents refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, .alpha.-monothioglycerol and sodium thio sulfate; low molecular weight polypeptides (i.e. <10 residues); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; polysaccharides such as dextran. Stabilizers may be present in the range from 0.1 to 10,000 weights per part of weight therapeutic agent.

Non-ionic surfactants or detergents (also known as "wetting agents") may be added to help solubilize the therapeutic agent as well as to protect the therapeutic protein against agitation-induced aggregation. Suitable non-ionic surfactants include, but are not limited to, polysorbates (20, 80, etc.), polyoxamers (184, 188 etc.), PLURONICS™, polyols, polyoxyethylene sorbitan monoethers (TWEEN™-20, TWEEN™-80, etc.). Non-ionic surfactants may be present in a range of about 0.05 mg/ml to about 1.0 mg/ml, preferably about 0.07 mg/ml to about 0.2 mg/ml.

Additional miscellaneous excipients include, but are not limited to, bulking agents, (e.g. starch), chelating agents (e.g. EDTA), antioxidants (e.g., ascorbic acid, methionine, vitamin E), and cosolvents.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin micropheres, microemulsions, nano-particles and nanocapsules) or in macroemulsions. Such techniques are disclosed in Remington: The Science and Practice of Pharmacy 20th edition (2000).

The form of the pharmaceutical compositions, the route of administration, the dosage and the regimen depend upon the condition to be treated, the severity of the illness, the age, weight, and sex of the patient, etc.

The doses used for the administration can be adapted as a function of various parameters, and in particular as a function of the mode of administration used, of the relevant pathology, or alternatively of the desired duration of treatment.

The pharmaceutical compositions of the invention can be formulated for a topical, oral, parenteral, intranasal, intravenous, intramuscular, subcutaneous or intraocular administration and the like.

Preferably, the pharmaceutical formulation is a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

Sterile injectable solutions may be prepared by incorporating the active compounds in the required amount in the appropriate solvent with various of the other ingredients enumerated above, as required, followed by filtered sterilization. In the case of sterile powders for the preparation of sterile injectable solutions, the preferred methods of preparation are vacuum-drying and freeze-drying techniques which yield a powder of the active ingredient plus any additional desired ingredient from a previously sterile- filtered solution thereof.

In addition to the compositions formulated for parenteral administration, such as intravenous or intramuscular injection, other pharmaceutically acceptable forms include, e.g. tablets or other solids for oral administration; time release capsules; and any other form currently used.

The pharmaceutical composition of the invention may comprise one or several antibodies of the invention.

The pharmaceutical composition may further comprise one or several additional active compounds. Examples of additional active compounds include, but are not limited to, chemotherapeutic drug, antibiotics, antiparasitic agents, antifungal agents or antiviral agents.

The amount of antibody of the invention which will be effective in the treatment of a particular disorder or condition will depend on the nature of the disorder or condition, and can be determined by standard clinical techniques.

In a preferred embodiment, each dose may range from about 0.1 mg to about 25 mg per kilogram of body weight of antibody, or more preferably, from about 1 mg to about 10 mg per kilogram body weight.

The dosing schedule for administration may vary form once a month to daily depending on a number of clinical factors, including the type of disease, severity of disease, and the subject's sensitivity to the therapeutic agent.

### Therapeutic applications

The present invention further relates to an antibody, nucleic acid, vector, host cell or pharmaceutical composition of the invention for use as a medicament, particularly in the prevention or treatment of a disease associated with sPLA2-GIB.

The present invention also relates to the use of an antibody, nucleic acid, vector, host cell or pharmaceutical composition of the invention as a medicament, for the treatment of a disease. The invention also relates to the use of an antibody, nucleic acid, vector, host cell or pharmaceutical composition of the invention for the manufacture or preparation of a medicament for treating a disease in a subject.

In particular, the present invention relates to a method of treating a disease in a subject, comprising administering to a subject in need thereof an effective amount of an antibody, nucleic acid, vector, host cell or composition of the invention.

As used herein, the term "subject" or "patient" refers to any mammal, preferably a human being.

The effective amount may be a therapeutically or prophylactically effective amount. A "therapeutically effective amount" refers to an amount effective, at dosages and for periods of time necessary, to achieve the desired therapeutic or prophylactic result. The therapeutically effective amount of an antibody or composition of the invention may vary according to factors such as the disease state, age, sex, and weight of the individual, and the ability of the antibody or composition, to elicit a desired response in the individual. A therapeutically effective amount encompasses an amount in which any toxic or detrimental effects of the antibody or composition are outweighed by the therapeutically beneficial effects. A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount would be less than the therapeutically effective amount.

The invention may be used to prevent or treat any disease or disorder associated with some degree of undesirable PLA2-GIB activity.

In a particular embodiment, the invention may be used to prevent or treat any disease related to an inappropriate (e.g., defective or improper) immune response, particularly to an inappropriate CD4 T cell activity, as well as any disease where an increased immunity may ameliorate the subject condition. These diseases are sometime referred to as "immune disorders" or "immunodeficiencies" in the present application. This includes particularly immunodefective situations caused by viral infection or pathogenic infection, or cancers.

As used herein, the term "treatment" or "treat" refers for instance to any clinical intervention in an attempt to alter the natural course of the individual being treated, and can be performed either for preventive or curative purpose. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, compositions and methods of the invention are used to delay development of a disease or disorder or to slow the progression of a disease or disorder.

Examples of diseases that can benefit from such treatment are all diseases with an immunodeficiency such as HIV-mediated immunodeficiency. In this regard, in a particular embodiment, the invention is directed to methods for treating an immunodeficiency or an associated disorder in a subject in need thereof, comprising administering an antibody, nucleic acid, vector, host cell or composition as defined above to said subject.

In another particular embodiment, the invention is directed to an antibody, nucleic acid, vector, host cell or composition as defined above for use for treating an immunodeficiency or an associated disorder in a subject in need thereof.

Immunodeficiencies and associated disorders designate any condition or pathology characterized by and/or caused by a reduced immune function or response in a subject. Immunodeficiencies may be caused by e.g., viral infection (e.g., HIV, hepatitis B, etc.), bacterial infection, cancer, or other pathological conditions. The terme "immunodeficiency-associated disorder" therefore designates any disease caused by or associated with an immunodeficiency. The invention is particularly suitable for treating immunodeficiencies related to CD4-T cells, and associated diseases.

In a particular embodiment, the invention relates to methods of treating HIV infection in a subject by administering an antibody, nucleic acid, vector, host cell or composition as defined above to said subject. In some embodiments the subject is an early HIV patient and the methods results in increasing the probability that the patient is a HIV controller. In some embodiments the subject is a patient with low immunoreconstitution after antiretroviral treatment and/or with severe idiopatic CD4 T lymphopenia (ICL). The invention also relates to a method for increasing CD4-T cell activity in a HIV-infected subject by administering a compound of formula A to said subject.
The invention may be used to treat subjects at an early stage of the infection, to prevent or reduce occurrence, extent, or duration of an immunodeficiency. Typically, they can be administered immediately upon detection of an infectious disease, and prior to appearance of clinical signs. At very early during infection by HIV, the invention can lead the patients toward a HIV controller status. The invention may also be used later in infected subjects, either alone or in combination with antiviral agent(s). In such regimen, the invention can accelerate the recovery of CD4 T lymphocytes and the restoration of their functions. Accordingly, in a particular embodiment, the invention comprises simultaneously, separately or sequentially administering to subject having a viral infection (i) an antiviral agent and (ii) an antibody, nucleic acid, vector, host cell or composition as defined above. Such protocol is particularly suited for treating HIV infected subjects, wherein an antibody of the invention is used in combination with antiretroviral therapy (e.g., HAART), allowing to reduce HAART or even to at least temporarily interrupt HAART treatment which is known for its severe detrimental effects.

The invention also provides methods for treating cancer by increasing an immune response in the subject, comprising administering an antibody, nucleic acid, vector, host cell or composition as defined above to said subject. The invention also provides methods of treating CD4 T cell-linked immunodeficiency associated with cancer in a subject by administering an antibody, nucleic acid, vector, host cell or composition as defined above to said subject.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

The following examples are given for purposes of illustration and not by way of limitation.

### EXAMPLES

### 1- Generation and characterization of the anti-sPLA2-GIB antibody 14G9

### 1.1 - Generation of the #14G9 mAb

Briefly, mice were immunized with recombinant human sPLA2-GIB, and generated mAb were screened for their inhibitory capacity and respective affinity for sPLA2-GIB versus pro-sPLA2-IB.

### 1.2 - Characterization of #14G9 mAb

### 1.2.1 - Affinity by competitive ELISA

Binding affinities of the antibodies to pro-sPLA2-IB and sPLA2-IB were measured by plate-based competition immunoassay. For the competition ELISA, briefly, constant amounts of antibody at different levels were premixed with serial dilutions of antigen protein, human sPLA2-IB or pro-sPLA2-IB, ranging from 0 to 10 µg/ml, and incubated for two hours at room temperature to reach pseudo-binding equilibrium between the antibody and antigen. These solutions were then transferred to 96-well sPLA2-IB or pro-sPLA2-IB pre-coated plates to allow the free-antibody in the mixtures to bind to plate-coated sPLA2-IB or pro-sPLA2-IB. The plates were typically coated with 30 to 100 ng of human sPLA2-IB or pro-sPLA2-IB protein in PBS solution overnight at 4° C followed by BSA nonspecific blocking. After washing off excess antibody in solution, plate-bound antibodies were detected with an HRP-conjugated goat anti-mouse IgG or IgA polyclonal antibody reagent and developed using colorimetric substrate. The dependency of the signals on the concentrations of antigen in solution was analyzed with a 4-parameter fit analysis using Prism™ software (Graph Pad) and reported as IC50.

### 1.2.2 - Affinity determination by SPR

The affinity constants (KD) of the pro-sPLA2-IB and sPLA2-IB binding to the #14G9 antibody were determined by surface kinetics on a real-time biosensor surface plasmon resonance assay (BIAcore™). More specifically, the affinity of the antibodies for human pro-sPLA2-IB and sPLA2-IB was measured using a BIAcore® 2000. The antibody was captured on an anti-mouse IgG surface and exposed to various concentrations of recombinant pro-sPLA2-IB and sPLA2-IB protein. Kinetic analysis using BIAevaluation™ software was performed to obtain the association and dissociation rate constants.

### 1.2.3 - Determination of specificity by indirect ELISA

Microplate wells were typically coated with 100 ng of recombinant human sPLA2-X, sPLA2-IB or pro-sPLA2-IB in PBS pH 7.5, overnight at 4°C. Sample wells were washed three times with PBS containing 0.05% Tween 20. After final washing, sample wells were treated with blocking solution containing 1% bovine serum albumin (BSA) in PBS buffer for 60 min at room temperature. Following washing with PBS containing 0.05% Tween 20, increasing amounts (10 ng/mL up to 3 µg/mL) of #14G9 mAb were added to antigen-coated wells. and incubated for 120 min at room temperature. Following washing with PBS containing 0.05% Tween 20, the binding of #14G9 mAb was detected with an HRP-conjugated goat anti-mouse IgG or IgA polyclonal antibody reagent and developed using colorimetric substrate. The dependency of the signals on the concentrations of antigen in solution was analyzed with a 4-parameter fit analysis using Prism™ software (Graph Pad) and reported as EC50.

### 1.2.4 - Inhibition of sPLA2-IB enzymatic activity

Inhibition of sPLA2-IB enzymatic activity by the #14G9 mAb was tested according to published procedures (Rouault et al. 2007). Briefly, recombinant human sPLA2-IB (0.1 nM) was incubated with increasing amount (0.1. 0.3. 1 and 3 µg) of a purified rabbit polyclonal immun-serum targeted towards human sPLA2-IB or of purified #14G9 antibody for 30 min at 37°C. sPLA2 enzymatic activity was then measured using [3H]-oleate-radiolabeled autoclaved E. coli membranes as phospholipid substrate in sPLA2 activity buffer. Reactions were stopped by addition of 300 µL of stop buffer, mixtures were then centrifuged at 10000g for 5 min, and the supernatants containing released [3H]-oleate were counted.

Results were confirmed using a commercial fluorimetric enzymatic activity assay. Recombinant human sPLA2-IB (3 nM) was incubated with increasing amount of purified #14G9 mAb for 120 min at room temperature, sPLA2 enzymatic activity was then measured using a selective fluorimetric method (AteroDX® sPLA2 Activity, Aterovax Paris, France). Results are expressed in Unit per mL of sample (U/mL), with one unit defined as the amount of sPLA2 enzyme which catalyzes the release of one nmol of product in one min. Limit of detection of the assay is 17 U/mL with upper linear analytical range of 232 U/mL and functional sensitivity (20%) is 21 U/mL. Average within-run variability and average intra-assay variability are 5.9% and 8.9%. respectively. Results of both methods were analyzed with a 4-parameter fit analysis using Prism™ software (Graph Pad) and reported as IC50.

### 1.3 - In vitro effect of the #14G9 mAb on the IL-7-induced nuclear translocation of phospho-STAT5 in CD4 T cells

### 1.3.1 - IL-7 induced nuclear translocation of phospho-STAT5

Human CD4 were purified using the RosetteSep isolation kit (Stemcell Technologies, #15062). CD4 T-cells were resuspended at 10⁶ cells/ml in RPMI 1640 medium (Lonza, Verviers, Belgium) supplemented with 5% foetal bovine serum (FBS), 50 mM HEPES pH 7.4, glutamine, penicillin, streptomycin and fungizone (complete medium) and equilibrated at least 2 h at 37°C in a 5% CO2 humidified atmosphere.

pSTAT5 nuclear translocation was followed using confocal microscopy. After equilibration, immobilized cells were plated on polylysine-coated glass slides before activation for 15 min with 2 nM IL-7. The cells were fixed in 4% PFA for 15 min at 37°C then 15 min at RT and permeabilized in 90% methanol/water solution at -20°C. pSTAT5 was then revealed by staining with rabbit anti-pSTAT5 (CST, #9359) labelled with donkey anti-rabbit-AlexaFluor 555 (Life Technologies, #A31572). For human CD4 T lymphocytes, cells were stained with mouse anti-human CD4 (eBioscience, #14-0042-82) labelled with goat anti-mouse-AlexaFluor 488 (Life Technologies, #A11029). For mice CD4 T lymphocytes cells were stained with rat anti-mouse CD4 (eBioscience, #14-0042-85) labelled with chicken anti-rat-AlexaFluor 488 (Life Technologies, #A21470). Images were acquired above the diffraction limit on an inverted laser scanning confocal microscope (LSM700, Zeiss) with a water-immersion apochromatic 40x/1.2 NA objective lens (Zeiss) or an oil-immersion plan-apochromatic 63x/1.4 NA objective lens (Zeiss). Images were acquired and analysed with the ZEN software (Zeiss).

### 1.3.2 - In vitro effect of #14G9 mAb on the inhibition by HIV-1 positive plasma of IL-7 induced phospho-STAT5 nuclear translocation in human CD4 T cells

For the dose-response experiment, increasing amounts of the #14G9 mAb, or isotypic isotype mAb, were firstly incubated for 25 min at room temperature, followed by 5 min at 37°C, together with recombinant sPLA2-IB or human plasma from one HIV-1 positive patient (3% dilution v/v in PBS). The equilibrated mixture was then added to CD4 T cells isolated from one healthy donor and the nuclear translocation of phospho-STAT5 was analyzed by confocal microscopy as described above. For the single dose experiment, 10 µg (final concentration) of #14G9 mAb, the non-inhibiting #6F7 mAb, or isotype control mAb, were firstly incubated for 25 min at room temperature, followed by 5 min at 37°C, together with human plasma from five different HIV-1 positive patients (3% dilution v/v in PBS). The equilibrated mixture was then added to CD4 T cells isolated from one healthy donor and the nuclear translocation of phospho-STAT5 was analyzed by confocal microscopy as described above.

### 1.4 - In vivo neutralization effect of the #14G9 mAb on the inhibition by sPLA2-GIB of IL-7 induced phospho-STAT5 nuclear translocation on CD4 T cells

30 C57BL/6J SOPF female mice (Charles River Laboratories) were kept in individually ventilated cages in SOPF conditions with sterile food and water provided ad libitum, and under day/night cycles of 12 hours, and were allowed to settle into their new environment for 5 days before the beginning of the experiments. All experimental procedures have received an approval from the local Animal Ethical Committee (CETEA 89, Institut Pasteur de Paris). The 30 animals were divided among 6 experimental groups (1 cage per group), which received different combinations of the test products by intraperitoneal injection. Group 1 received 1 mg (300 µL) of isotype control mAb at T0, then 100 µg (100 µL) of sPLA2-IB at T+2h, group 2 received 1 mg (300 µL) of #14G9 mAb at T0, then 100 µg (100 µL) of sPLA2-IB at T+2h, group 3 received 100 µg (100 µL) of sPLA2-GIB at T+2h, group 4 received PBS injection (100 µL) at T+2h, group 5 received received PBS injection (100 µL) at T0, and group 6 received 100 µg (100 µL) of sPLA2-GIB at T0. Mice were euthanized by cervical dislocation at different time points after onset of the study. Groups 1 to 4 were euthanized at T + 5h and groups 5 and 6 at T+ 48h. Immediately after euthanasia, spleens were harvested from the animals and kept at room temperature in 15 mL tubes containing 5mL of RPMI 5% until further processing. Mouse splenocytes were prepared with the gentleMACS Dissociator (program m_spleen_01) then mouse CD4 were isolated by negative selection using EasySep isolation kit (Stemcell Technologies, #19851). The nuclear translocation of phospho-STAT5 was analyzed by confocal microscopy as described above. Mann-Whitney test was used to compare medians of the groups.

### 1.5 - Sequences of the #14G9 mAb

Variable regions of the #14G9 mAb were sequenced according to classical procedures. Briefly, highly pure RNA was extracted from hybridoma cells and complementary DNA strand were synthesized using high-fidelity reverse-transcriptase. PCR products generated using high-fidelity PCR and degenerate primers targeting specifically cDNA encoding for antibody heavy chain and light chain were directly sequenced (double strand sequencing). cDNA sequences were analyzed and assembled. Peptidic sequences were translated and validated according to antibody structure.

### 2- Humanization of #14G9 antibody

### 2.1 - Humanization strategy and generation of #14G9 humanized variants

The #14G9 mouse antibody was humanized by grafting the three CDRs, as defined by the Kabat nomenclature, from the light chain variable region (VL) into a human germline VL that was as homologous as possible to the mouse antibody VL. Similarly, the three CDRs from the heavy chain variable region (VH) were grafted into a human germline VH that was as homologous as possible to the mouse antibody VH. In addition, a few amino acid residues in the framework regions of the selected human germline variable regions were changed to the amino acid residues that were present in the mouse variable regions (so called back mutations).

### 2.2 - Inhibition of sPLA2-GIB enzymatic activity by humanized antibodies

Inhibition of sPLA2-GIB enzymatic activity by the humanized antibodies #1A, #1B, #2A and #2B was tested according to published procedures (Rouault et al. 2007). Briefly, recombinant human sPLA2-GIB (0.05nM) was incubated with increasing amount (0.1. 0.3. 1 and 3 µg) of purified antibodies for 30 min at 37°C. sPLA2 enzymatic activity was then measured using [3H]-oleate-radiolabeled autoclaved E. coli membranes as phospholipid substrate in sPLA2 activity buffer. Reactions were stopped by addition of 300 µL of stop buffer, mixtures were then centrifuged at 10000g for 5 min, and the supernatants containing released [3H]-oleate were counted.

### 2.3 - Affinity determination of humanized antibodies by competitive ELISA

Binding affinities of the humanized antibodies to pro-sPLA2-GIB and sPLA2-GIB were measured by plate-based competition immunoassay. For the competition ELISA, briefly, constant amounts of antibody at different levels were premixed with serial dilutions of antigen protein, human sPLA2-GIB or pro-sPLA2-GIB, ranging from 0 to 10 µg/ml, and incubated for two hours at room temperature to reach pseudo-binding equilibrium between the antibody and antigen. These solutions were then transferred to 96-well sPLA2-GIB or pro-sPLA2-GIB pre-coated plates to allow the free-antibody in the mixtures to bind to plate-coated sPLA2-GIB or pro-sPLA2-GIB. The plates were typically coated with 30 to 100 ng of human sPLA2-GIB or pro-sPLA2-GIB protein in PBS solution overnight at 4° C followed by BSA nonspecific blocking. After washing off excess antibody in solution, plate-bound antibodies were detected with an HRP-conjugated goat anti-mouse IgG or IgA polyclonal antibody reagent and developed using colorimetric substrate. The dependency of the signals on the concentrations of antigen in solution was analyzed with a 4-parameter fit analysis using Prism™ software (Graph Pad) and reported as IC50.

### 2.4 - Differential Scanning Calorimetry of humanized antibodies

The thermal stability in PBS buffer of antibodies #1A, #1B, #2A and #2B was compared by Differential Scanning Calorimetry (DSC) on a Microcal VP-Capillary DSC system. All samples were shipped in dry ice, and thawed by overnight incubation at -20 °C, followed by ice incubation for 2 hours. After sample thawing, all samples were centrifuged (20.000 xg, 5 min, 4 °C), and had their protein content quantitated on a Nanodrop ND-1000 spectrophotometer with IgG analysis program, prior to DSC analysis. The pre-equilibration time was 3 min and the thermograms that followed were acquired between 20 and 110 °C with a scanning rate of 60 °C/hour, a filtering period of 25 sec and medium feedback. Prior to sample analysis, 5 buffer/buffer scans were measured to stabilize the instrument, and a buffer/buffer scan was performed between each protein/buffer scan.

### 2.5 - Analytical Size Exclusion Chromatography (SEC-HPLC) of humanized antibodies

Analytical Size Exclusion Chromatography (SEC-HPLC) was performed on a Shimadzu Prominence HPLC on a Superdex 200 increase 5/150 GL column (GE Healthcare). The column was previously equilibrated in PBS, at 0.25 mL/min, with the column oven set to 30 °C. All samples were centrifuged (20 000g, 5 min, 4°C), and had their protein content quantitated on a Nanodrop ND-1000 spectrophotometer with IgG analysis program, prior to SEC analysis. The isocratic program was set to inject about 15 µg of each sample, at 0.25 mL/min during 18 min. After SEC analysis, 280nm chromatogram was extracted from the raw data, and analysed by peak integration. A series of proteins from the GE Lifesciences Molecular Weight SEC Calibration kits were used to calibrate the column in the same conditions and buffer used during the sample analysis. The proteins used were: Aprotinin (6.500 Da), Ribonuclease A (13.700 Da), Carbonic Anhydrase (29.000 Da), Ovalbumin (44.000 Da), Conalbumin (75.000 Da) and Aldolase (158.000). Blue Dextran was used to determine the Void Volume of the column. Column calibration was done according to the Gel Filtration Calibration Kits instructions (GE Lifesciences).

### 3- Characterization of #2B1 and #2B2

### 3.1- SPR affinity determination of #2B, #2B1 and #2B2 antibodies

The affinity constants (KD) of the pro-sPLA2-GIB and sPLA2-GIB binding to the #2B, #2B1 and #2B2 antibodies were determined by surface kinetics on a real-time biosensor surface plasmon resonance assay (BIAcore™). More specifically, the affinity of the antibodies for human pro-sPLA2-IB and sPLA2-IB was measured using a BIAcore® 2000. The antibody was captured on an anti-mouse IgG surface and exposed to various concentrations of recombinant pro-sPLA2-IB and sPLA2-IB protein. Kinetic analysis using BIAevaluation™ software was performed to obtain the association and dissociation rate constants.

### 3.2 - Differential Scanning Calorimetry of #2B1 and #2B2 humanized antibodies

The thermal stability in PBS buffer of the #2B1 and #2B2 antibodies was compared by Differential Scanning Calorimetry (DSC) as described above.

### 3.3- Analytical Size Exclusion Chromatography (SEC-HPLC) of #2B1 and #2B2 humanized antibodies

Analytical Size Exclusion Chromatography (SEC-HPLC) analysis of the #2B1 and #2B2 was performed as described above.

### 3.4 - Capillary Isoelectric Focusing analysis of #2B1 and #2B2 humanized antibodies

The iso-electric point and relative distribution of charge variants of the #2B, #2B1 and #2B2 were determined by imaged capillary isoelectric focusing (icIEF) analysis using Pharmalyte pH3-10 (broad range suitable for most mAbs) in the presence of urea at a final protein concentration of 0.2 mg/mL. Two pI markers bracketing the expected pI of the sample were used for calibration. Data read-out was measured as absorbance (at 280 nm) and expressed in an electropherogram. pI values were calculated using the Compass and Empower 3 software and peak integration was performed in Empower 3.

### 3.5 -Binding of #2B, #2B1 and #2B2 humanized antibodies to human FcγRs

Monomeric IgG binding was tested by incubating the #2B, #2B1 and #2B2 antibodies (100 ng/mL) with 1 × 10⁵ CHO cells stably expressing human FcγRs for 30 min on ice. The cells were then washed, detached, fixed, and analyzed by flow cytometry (FACSCalibur; BD Biosciences). Bound mAbs were detected using a PE-conjugated goat anti-human IgG F(ab')2 fragment at 0.5 mg/ml (Jackson Laboratories; Cat# 109-096-006).

Antibody-antigen immune complex were generated by co-incubation of 10 µg/ml of humanized antibodies and 5 µg/ml of sPLA2-GIB for 30 min at 37 °C. Immune complexes were then incubated with 1 × 10⁵ CHO cells stably expressing human FcyRs for 30 min on ice. The cells were then washed, detached, fixed, and analyzed by flow cytometry (FACSCalibur; BD Biosciences). Bound mAbs were detected using a PE-conjugated goat anti-human IgG F(ab')2 fragment at 0.5 mg/ml (Jackson Laboratories; Cat# 109-096-006).

The residual binding of antigen on antibody-sensitized cells was tested by firstly incubating the #2B, #2B1 and #2B2 antibodies (10 µg/mL) with 1 × 10⁵ CHO cells stably expressing human FcγRs for 30 min on ice. After washing, the sPLA2-IB biotinylated antigen (5 µg/mL) was added for 30 min on ice. The cells were then washed, detached, fixed, and analyzed by flow cytometry. Bound biotinylated antigen was detected using an avidin-FITC-conjugate. All data were analyzed with Flow Cytometry Analysis Software (FlowJo).

### 4- Biological properties of #2B2 humanized antibody

### 4.1 - In vitro effect of the #2B mAb on the IL-7-induced nuclear translocation of phospho-STAT5 in CD4 T cells

Increasing amounts of the #14G9 or #2B antibodies were first incubated for 25 min at room temperature, followed by 5 min at 37°C, together with 75 nM recombinant sPLA2-GIB or human plasma from one HIV-1 positive patient (3% dilution v/v in PBS). The equilibrated mixture was then added to CD4 T cells isolated from one healthy donor and the nuclear translocation of phospho-STAT5 was analyzed by confocal microscopy as described above. Dose dependent curves were analyzed with a 4-parameter fit analysis using Prism™ software (Graph Pad) and reported as EC50.

### 1.4 - In vivo neutralization effect of the #2B2 on the inhibition by sPLA2-IB of IL-7 induced phospho-STAT5 nuclear translocation on CD4 T cells

Eighteen 7-weeks old C57BL/6J SOPF male mice (Charles River Laboratories) were kept in individually ventilated cages in SOPF conditions with sterile food and water provided ad libitum, and under day/night cycles of 12 hours, and were allowed to settle into their new environment for 5 days before the beginning of the experiments. All experimental procedures have received an approval from the local Animal Ethical Committee (CETEA 89, Institut Pasteur de Paris). The 18 animals were divided among 5 experimental groups (1 cage per group), which received different combinations of the test products by peritoneal injection. Group 1 received PBS injection (350µL) at T0, then PBS injection (100µL) at T+2h, group 2 received PBS injection (350 µL) at T0, then 100µg (100 µL) of sPLA2-GIB at T+2h, group 3 received 2 mg (350 µL) of isotypic control mAb at T0, then 100µg (100 µL) of sPLA2-GIB at T+2h, group 4 received 1 mg (350 µL) of #2B2 humanized mAb at T0, then 100µg (100 µL) of sPLA2-IB at T+2h, group 5 received 2 mg (350µL) of #2B2 humanized mAb at T0, then 100µg (100µL) of sPLA2-IB at T+2h. Mice were euthanized by cervical dislocation at T + 5h after onset of the study. Immediately after euthanasia, spleens were harvested from the animals and immediately processed. Mouse splenocytes were prepared with the gentleMACS Dissociator, then mouse CD4 were isolated by negative selection using EasySep isolation kit (Stemcell Technologies, #19851). The nuclear translocation of phospho-STAT5 was analyzed by confocal microscopy as described above. Mann-Whitney test was used to compare medians of the groups.

### RESULTS

### 5- Generation and characterization of the anti-sPLA2-IB 14G9 antibody

### 5.1 - Generation and characterization of the #14G9 mAb

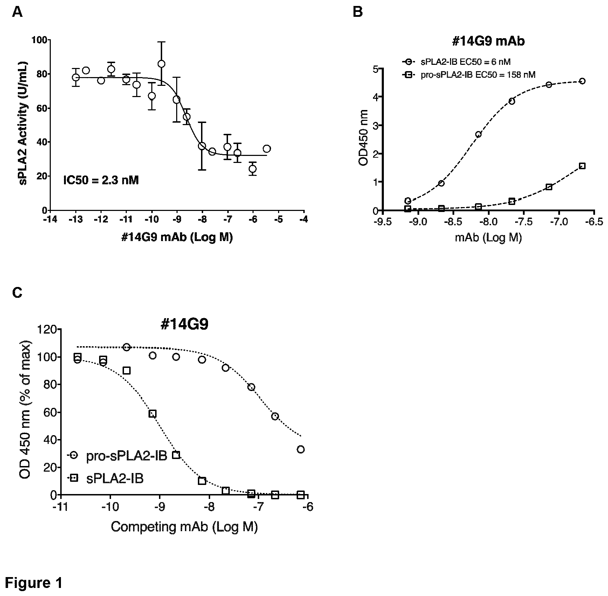

Briefly, mice were immunized with recombinant human sPLA2-IB, and generated mAb were screened for their inhibitory capacity and respective affinity for sPLA2-IB versus pro-sPLA2-IB. #14G9 mAb was shown to inhibit sPLA2-IB enzymatic activity with an estimated EC50 of 2.3 nM (Figure 1A), to be highly specific for sPLA2-GIB vs pro-sPLA2-GIB (Figure 1B and 1C). In competing ELISA, IC50 were estimated to be 108.6 nM and 1.0 nM for pro-sPLA2-GIB and sPLA2-GIB, respectively. The affinity constant (KD) of the pro-sPLA2-GIB and sPLA2-GIB binding to #14G9 was determined by surface kinetics on a real-time biosensor surface plasmon resonance assay (BIAcore™). In these experiments, Kd were estimated to be 174 nM and 0.34 nM for pro-sPLA2-IB and sPLA2-IB, respectively.

### 5.2 - In vitro effect of #14G9 mAb on the inhibition by plasma from HIV-1 infected patients of IL-7 induced phospho-STAT5 nuclear translocation in CD4 T cells

Recently, the sPLA2-IB enzyme was identified as playing a crucial role in the mechanism behind the unresponsiveness of CD4 T lymphocytes in viremic HIV-infected patients. In particular, it was demonstrated that sPLA2-IB is the protein responsible for the chronic activation of CD4 T lymphocytes, which drives them into an aberrant state of activation differentiation which renders them refractory to certain physiological signals such as those delivered by interleukin-7 (THEZE Jacques et al. 2015).

The ability of the #14G9 mAb to counterbalance the inhibitory effect of plasma isolated from HIV-1 infected patients on the IL-7 induced phospho-STAT5 nuclear translocation in CD4 T cells was tested by incubating plasmas isolated from HIV-1 viremic patients with the #14G9 mAb.

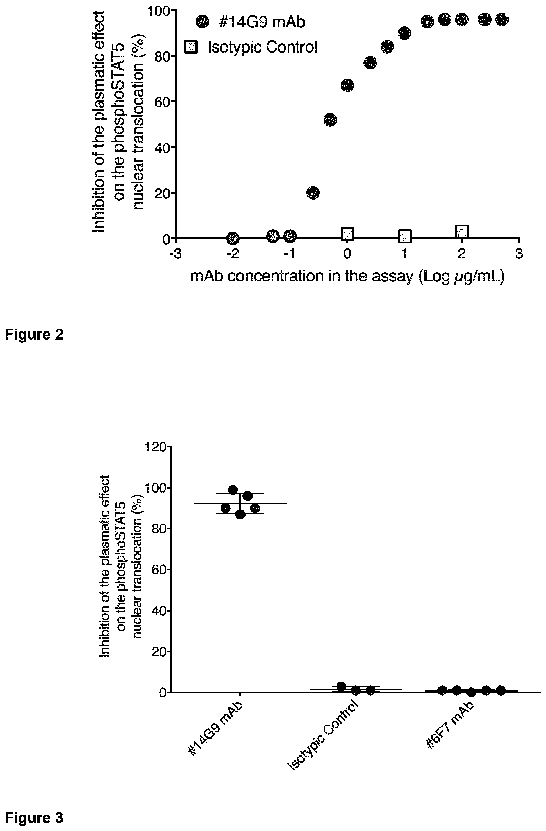

As depicted in Figure 2, increasing amounts of the #14G9 mAb led to a dose dependent restoration of the ability of plasmas from viremic patients to inhibit the nuclear translocation of phosphoSTAT5 induced by IL-7 in human CD4 T cells isolated from one healthy donor. This dose-response restoration effect was not observed with an isotype control mAb. These results were reproduced in 5 successive experiments, on CD4 T cells isolated from 5 different healthy donors using a single dose (10 µg) of the #14G9 mAb, or 10 µg of the non-inhibitory #6F7 mAb or isotype control mAb (Figure 3).

### 5.3 - In vivo effect of #14G9 mAb on the inhibition of phospho-STAT5 nuclear translocation by sPLA2-IB

The ability of the #14G9 mAb to counterbalance *in vivo* the inhibitory effect of the injection of sPLA2-IB on the IL-7 induced phospho-STAT5 nuclear translocation in CD4 T cells was tested by first injecting 1 mg of the #14G9 mAb in mice, followed by the injection of 100 µg of sPLA2-IB. Nuclear translocation of phospho-STAT5 in CD4 T cells isolated from splenocytes of the treated mice was then analyzed by confocal microscopy.

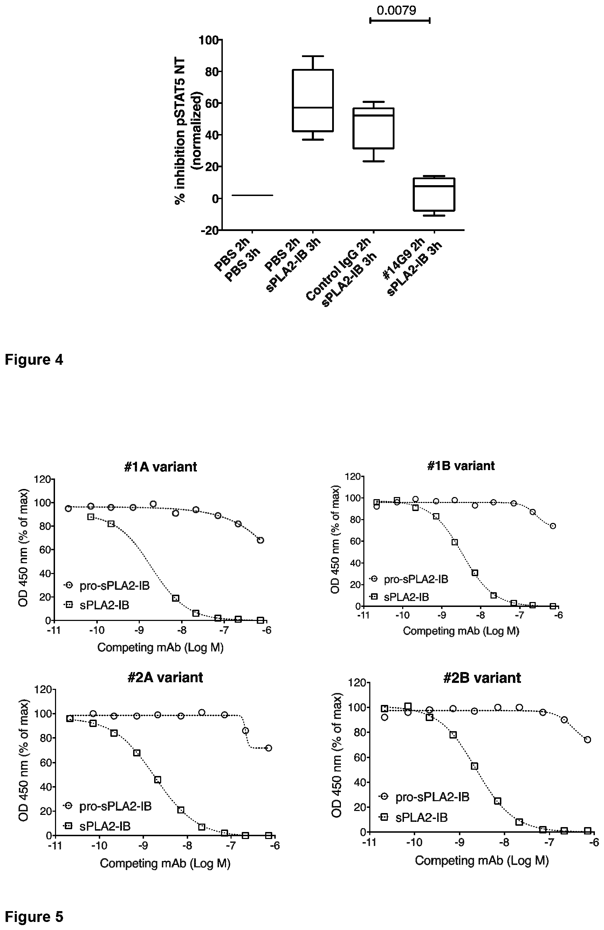

As depicted in Figure 4, the injection of 1 mg of sPLA2-IB in mice led to a 60% inhibition of the nuclear translocation of phosphoSTAT5 in CD4 T cells isolated from splenocytes. This effect was fully abolished by the prior injection of 1 mg of the #14G9 mAb, with no significant effect observed in control injections.

### 5.4 - Sequences of #14G9 mAb variable regions

The variable regions of light and heavy chains of the #14G9 mAb were sequenced and are presented below.
>14G9-VL (SEQ ID NO: 8) 
GACATTGTGATGACCCAGTCTCACAAACTCATGTCCACATCAGTAGGAGACAGGGTCAG
CATCACCTGCAAGGCCAGTCAGGATGTGAGTACTGCTGTAGCCTGGTATCAAGAAAAAC
CAGGTCAATCTCCTAAACTACTGATTTACTGGGCATCCACCCGGCACACTGGAGTCCCT
AATCGCTTCACAGGCAGTAGATCTGGGACAGATTATACTCTCACCATCAGCAGTGTGCA
GGCTGAAGACCTGGCACTTTATTACTGTCAGCAAGATTATAGCACTCCTCCTACGTTCG
 GTGCTGGGACCAAGCTGGAGCTGAAACGGGCTGATGCTGCACCAACTGTATCCATC
>14G9-VL (SEQ ID NO: 2) 
DIVMTQSHKLMSTSVGDRVSITCKASQDVSTAVAWYQEKPGQSPKLLIYWASTRHTGVP
NRFTGSRSGTDYTLTISSVQAEDLALYYCQQDYSTPPTFGAGTKLELKRADAAPTVSI
>14G9-VH (SEQ ID NO: 9) 
CAGGTCCAACTGCAACAGCCTGGGGCTGAGCTGGTGAAGCCTGGGGCTTCAGTGAAGCT
GTCCTGCAAGGCTTCTGGCTACACCTTCACCAACTACTGGATACACTGGGTGAAGCGGA
GGCCTGGACAAGGCCTTGAATGGATTGGTAATATTGACCCTTCTGATACTAGAACTCAC
TACAATCAAAAGTTCAAGGACAAGGCCACATTGGCTGTAGACAAATCCTCCAGCACAGC
CTACATGCAGCTCAGCAGCCTGACATCTGAGGACTCTGCGGTCTTTTACTGTGCAAGAC
AGACCCTCTACTATGAGGCCTTGGACTACTGGGGCCAAGGCACCACTCTCACAGTCTCC
TCAGCCAAAACGACACCCCCATCTGTCTATCCACTGGG
>14G9-VH (SEQ ID NO: 3)

QVQLQQPGAELVKPGASVKLSCKASGYTFTNYWIHWVKRRPGQGLEWIGNIDPSDTRTH
YNQKFKDKATLAVDKSSSTAYMQLSSLTSEDSAVFYCARQTLYYEALDYWGQGTTLTVS
SAKTTPPSVYPL

In the great majority of antibodies, the conserved VH/VL interface is mainly composed of hydrophobic interactions between Kabat positions H37, H45, H47, H89, H91, H100B and H103 in heavy chain framework residues and L36, L44, L46, L87, L96 and L98 in light chain framework residues, and of hydrogen bonding between H39 and L38.

A striking and unique feature of #14G9 monoclonal antibody is the very unusual presence of an Arg at Kabat position H39 and a Glu at Kabat position L38 instead of the usual and highly conserved Gln at both positions. In 14G9 the hydrogen-bonding interaction between Gln H39 and Gln L38 is replaced with an electrostatic attraction between Arg H39 and Glu L38.

### 6- Humanization of the #14G9 mouse monoclonal antibody

### 6.1 - Humanization strategy and generation of humanized antibodies

The #14G9 mouse antibody was humanized by grafting the three CDRs, as defined by the Kabat nomenclature, from the light chain variable region (VL) into a human germline VL that was as homologous as possible to the mouse antibody VL. Similarly, the three CDRs from the heavy chain variable region (VH) were grafted into a human germline VH that was as homologous as possible to the mouse antibody VH. In addition, a few amino acid residues in the framework regions of the selected human germline variable regions were changed to the amino acid residues that were present in the mouse variable regions (so called back mutations). Based on information on the structure of immunoglobulin variable regions, these few residues in the framework regions were identified as having key roles in maintaining the CDRs in the right conformation or in VH/VL packing and thus retained in humanized version A and B. For the design of CDR-grafted versions of #14G9 VH, two human germlines, IGHV1-46*01 and IGHV5- 10-1*01 were selected. Human germline IGHV1-46*01 was selected because it exhibited the best overall identity across the whole V gene with mouse 14G9 VH with 66.3%. Human germline IGHV5- 10-1*01 was selected because although it has less overall identity with 14G9 VH (58.2%) it has a different homology pattern across the whole 14G9 VH from IGHV1-46*01. For the design of CDR-grafted versions of 14G9 VL two different human Kappa germlines, IGKV1- 39*01 and IGKV3-15*01 were selected. IGKV1-39*01 was selected because it has the highest homology across the whole V gene with mouse 14G9 VL with 67.4% identity. IGKV3-15*01 despite a lower percentage identity (62.1%) was selected because it has different identity pattern within CDRs with #14G9 VL from IGKV1-39*01.

The design of the humanized versions took into account the unusual presence of Arg H39 and Glu L38 at the VH/VL interface. In version A of CDR-grafted VH and VL sequences, the parental mouse residues at H39 (Arg) and L38 (Glu) were conserved, respectively. In version B, the parental residues at Kabat positions H39 and L38 were substituted with Gln (the human germline counterpart residue) in order to recreate the highly conserved hydrogen bonding interaction. Consequently, for each human germline (2 for the VH and 2 for the VL) used for the design of CDR-grafted #14G9, 2 versions (A and B) were constructed. Version A had Arg H39 (VH) or Glu L38 (VL) whereas version B had Gln at both positions. Humanized version A of VH was only paired with humanized version A of VL and the same principle goes for humanized version B. The increase in percentage of sequence identity (i.e. percentage of humanness) across the whole V genes between the mouse #14G9 hybridoma and the CDR-grafted versions is presented in the table 1. The details of the differences in sequences across the V gene between the humanized versions and the corresponding immunoglobulin variable human germlines are presented in table 2.

### 6.2 - Inhibition of sPLA2-IB enzymatic activity by humanized antibodies

The ability of the different humanized antibodies to inhibit sPLA2-IB enzymatic activity was tested by incubating radiolabeled membranes with recombinant human sPLA2-IB in the presence of increasing amounts of purified monoclonal antibodies. Results are expressed as percentage of inhibition of enzymatic activity observed with the highest concentration of mAb tested and are presented in Table 3. As presented, the #2B is the mAb with the highest potency of inhibiting sPLA2-GIB.

### 6.3 - Affinity determination of humanized antibodies by competitive ELISA

The respective binding affinities of the humanized antibodies to human pro-sPLA2-GIB and human sPLA2-GIB were measured by plate-based competition immunoassay. Briefly, constant amounts of antibody at different levels were premixed with serial dilutions of antigen proteins to reach pseudo-binding equilibrium between the antibody and antigen. These solutions were then transferred to antigen pre-coated plates to allow the free-antibody in the mixtures to bind to plate-coated sPLA2-GIB or pro-sPLA2-GIB. Plate-bound antibodies were detected with an HRP-conjugated polyclonal antibody. Examples of the titration curves obtained for the different variants are presented in Figure 5. Data were fitted with a 4PL logistic non-linear regression model and the relative IC50 values were estimated from the model (Table 4).

### 6.4 - Differential Scanning Calorimetry of humanized antibodies

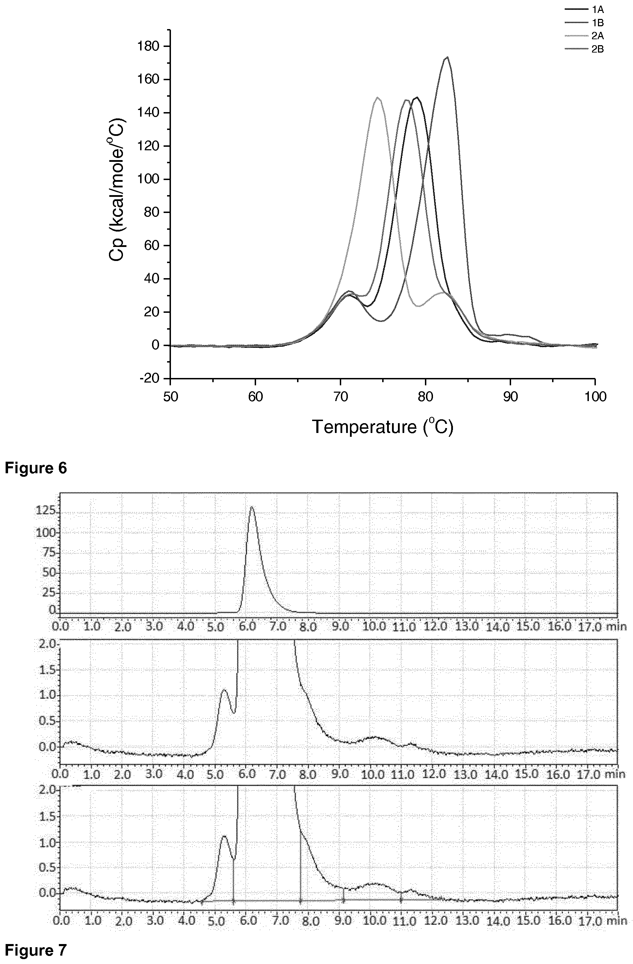

Normalized thermal denaturation of the different tested antibodies are presented in Figure 6, and DSC parameters in Table 5. Tm are denaturation/melting temperatures. T onset are the temperatures at which the unfolding transition begins. T1/2 are the width of the transition at half height of the peak. ΔH are the calorimetric enthalpy of unfolding and reflect the disruption of intramolecular interactions of the protein. Total Area are the entire enthalpy of unfolding (total area under the thermogram) and reflect thermodynamic stability. According to the superimposition of all thermograms (Figure 6), #1B is the one with better behavior by DSC. The first transition, about 70-72°C should correspond to CH2 domain. The last transition, about 82-83°C should correspond to CH3, and the highest peak corresponds to Fab transition. In #2A, the Fab transition is on top of the CH2, all the other variants have the Fab transition on top of CH3 transition, and at least on #2B, the shoulder on the right end of the Fab transition should correspond to the CH3 transition.

### 6.5 - Analytical Size Exclusion Chromatography (SEC-HPLC) of humanized antibodies

The humanized #1A, #1B, #2A and #2B antibodies were analyzed by SEC-HPLC in PBS buffer. A representative chromatogram for #2B is presented in figure 7 and results are summarized in Table 6. As presented, all variants display an apparent molecular weight close to 160 kDa, indicating monomeric conformation and absence of aggregation in the tested conditions.

### 6.6 - #2B sequences

Based on the inhibitory, binding and biophysical properties described above, the #2B antibody was selected for further optimization. The heavy chain of variant #2B displays 81.60% identity (80/98) with the IGHV1-46*01 human germline. The light chain of #2B displays 86.3% identity (82/95) with the IGKV3-15*01 germline. Overall % of identity of #2B is 83.94% with the human germlines of reference.

The sequences of the heavy and light chains of the #2B are presented below:

### Heavy chain

> 3352.VH 2B (SEQ ID NO: 11)

gcggccgccatgaattttggactgaggctgattttcctggtgctgaccctgaaaggcgtccagt
gtcaagtccagctcgtccagagcggggcggaagtcaagaagccgggggcgagcgtgaaagtctcgtgcaa
ggcgagcggatatacgttcacgaactactggattcactgggtccggcaagcgccggggcaggggctggag
tggatcgggaacatcgacccgtcggacacgcggacgcactacaaccagaagttcaaggaccgggcgacgc
tgaccgtcgacaagagcacgagcacggcgtacatggagctgtcgagcctgcggagcgaggacacggccgt
ctactactgcgcgcggcagacgctgtactacgaggcgctggactactggggcaggggacgctcgtcacg
gtctcgagcgctagcacaaaggccctagtgtgtttcctctggctccctcttccaaatccacttctggtg
gcactgctgctctgggatgcctggtgaaggattactttcctgaacctgtgactgtctcatggaactctgg

tgctctgacttctggtgtccacactttccctgctgtgctgcagtctagtggactgtactctctgtcatct
gtggtcactgtgccctcttcatctctgggaacccagacctacatttgtaatgtgaaccacaaaccatcca
acactaaagtggacaaaagagtggaacccaaatcctgtgacaaaacccacacctgcccaccttgtcctgc
ccctgaactgctgggaggaccttctgtgtttctgttcccccccaaaccaaaggataccctgatgatctct
agaacccctgaggtgacatgtgtggtggtggatgtgtctcatgaggaccctgaggtcaaattcaactggt
acgtggatggagtggaagtccacaatgccaaaaccaagcctagagaggaacagtacaattcaacctacag
agtggtcagtgtgctgactgtgctgcatcaggattggctgaatggcaaggaatacaagtgtaaagtctca
aacaaggccctgcctgctccaattgagaaaacaatctcaaaggccaagggacagcctagggaaccccagg
tctacaccctgccaccttcaagagaggaaatgaccaaaaaccaggtgtccctgacatgcctggtcaaagg
cttctacccttctgacattgctgtggagtgggagtcaaatggacagcctgagaacaactacaaaacaacc
ccccctgtgctggattctgatggctctttctttctgtactccaaactgactgtggacaagtctagatggc
agcaggggaatgtcttttcttgctctgtcatgcatgaggctctgcataaccactacactcagaaatccct
gtctctgtctcccgggaaatgatagtaaaagctttga

### Light chain

> 3352.VL2.B (SEQ ID NO: 10) 
Gcggccgccatgaattttggactgaggctgattttcctggtgctgaccctgaaaggcgtccagt
gtgagatcgtgatgacgcagtcgccggcgacgctgagcgtgagtccgggggagcgggcgacgctgagctg
caaggcgtcgcaggacgtgagcacggccgtcgcgtggtatcaacagaagccggggcaagcgccgcggctg
ctgatctactgggcgagcacgcggcacacgggcatcccggcgcgattctcggggagccggagcgggacgg
agtacacgctgacgatctcgtcgctgcaaagcgaggacttcgccgtctactactgccagcaggactactc
gacgccgccgacgttcgggggggtaccaaggtcgagatcaaacgtacggtcgcggcgccttctgtgttc
atttttcccccatctgatgaacagctgaaatctggcactgcttctgtggtctgtctgctgaacaacttct
accctagagaggccaaagtccagtggaaagtggacaatgctctgcagagtgggaattcccaggaatctgt
cactgagcaggactctaaggatagcacatactccctgtcctctactctgacactgagcaaggctgattac
gagaaacacaaagtgtacgcctgtgaagtcacacatcaggggctgtctagtcctgtgaccaaatccttca
ataggggagagtgctgatagtaaaagctttga
> Heavy chain (SEQ ID NO: 5) 
QVQLVQSGAEVKKPGASVKVSCKASGYTFTNYWIHWVRQAPGQGLEWIGNIDPSDTRTHYNQKF
KDRATLTVDKSTSTAYMELSSLRSEDTAVYYCARQTLYYEALDYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTYICNV
NHKPSNTKVDKRVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGK
> Light chain (SEQ ID NO: 4)

EIVMTQSPATLSVSPGERATLSCKASQDVSTAVAWYQQKPGQAPRLLIYWASTRHTGIPARFSG
SRSGTEYTLTISSLQSEDFAVYYCQQDYSTPPTFGGGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEVTHQGLSSP
VTKSFNRGEC

### 7- Characterization of #2B1 and #2B2 humanized variants

### 7.1 - Strategy for point mutations abolishing CDC and ADCC

The humanized #2B antibody was further optimized by introducing specific point mutations known to abolish both ADCC and CDC functions in the heavy chain constant region of the mAb.

The sequences of the resulting antibodies #2B1 (double mutations L234A & L235E) and #2B2 (triple mutation L234A, L235E and P331S) are presented below:

### 2B1 (mutations L234A & L235E)

### Heavy chain

SEQ ID NO: 12

gcggccgccatgaattttggactgaggctgattttcctggtgctgaccctgaaaggcgtccagt
gtcaagtccagctcgtccagagcggggcggaagtcaagaagccgggggcgagcgtgaaagtctcgtgcaa
ggcgagcggatatacgttcacgaactactggattcactgggtccggcaagcgccggggcaggggctggag
tggatcgggaacatcgacccgtcggacacgcggacgcactacaaccagaagttcaaggaccgggcgacgc
tgaccgtcgacaagagcacgagcacggcgtacatggagctgtcgagcctgcggagcgaggacacggccgt
ctactactgcgcgcggcagacgctgtactacgaggcgctggactactgggggcaggggacgctcgtcacg
gtctcgagcgctagcacaaagggccctagtgtgtttcctctggctccctcttccaaatccacttctggtg
gcactgctgctctgggatgcctggtgaaggattactttcctgaacctgtgactgtctcatggaactctgg
tgctctgacttctggtgtccacactttccctgctgtgctgcagtctagtggactgtactctctgtcatct
gtggtcactgtgccctcttcatctctgggaacccagacctacatttgtaatgtgaaccacaaaccatcca
acactaaagtggacaaaagagtggaacccaaatcctgtgacaaaacccacacctgcccaccttgtccggc
gcctgaagcggaaggaggaccttctgtgtttctgttcccccccaaaccaaaggataccctgatgatctcg
cgaacccctgaggtgacatgtgtggtggtggatgtgtctcatgaggaccccgaagtcaaatttaattggt
atgtcgacggcgtcgaggtgcataatgccaaaaccaagcctagagaggaacagtacaattcaacctacag
agtcgtcagtgtgctgactgtgctgcatcaggattggctgaatggcaaggaatacaagtgtaaagtctca
aacaaggccctgcctgctccaattgagaaaacaatctcaaaggccaagggacagcctagggaaccccagg
tctacaccctgccaccttcaagagaggaaatgaccaaaaaccaggtgtccctgacatgcctggtcaaagg

cttctacccttctgacattgctgtggagtgggagtcaaatggacagcctgagaacaactacaaaacaacc
ccccctgtgctggattctgatggctctttctttctgtactccaaactgactgtggacaagtctagatggc
agcaggggaatgtcttttcttgctctgtcatgcatgaggctctgcataaccactacactcagaaatccct
gtctctgtctcccgggaaatgataagctttga

### Light chain

same as 2B (see SEQ ID NO: 10)
> Heavy chain (SEQ ID NO: 6) 
QVQLVQSGAEVKKPGASVKVSCKASGYTFTNYWIHWVRQAPGQGLEWIGNIDPSDTRTH
YNQKFKDRATLTVDKSTSTAYMELSSLRSEDTAVYYCARQTLYYEALDYWGQGTLVTVSSASTK
GPSVFPLAPSSKSTSGGTAALGCLVKDYFPEPVTVS
WNSGALTSGVHTFPAVLQSSGLYSLSSVVTVPSSSLGTQTYICNVNHKPSNTKVDKRVE
PKSCDKTHTCPPCPAPEAEGGPSVFLFPPKPKDTLMISRTPEVTCVVVDVSHEDPEVKFNWYVD
GVEVHNAKTKPREEQYNSTYRVVSVLTVLHQDWLNG
KEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQK
SLSLSPGK
> Light chain
   same as 2B (see SEQ ID NO: 4)
**2B2** (mutations L234A, L235E and P331S)

### Heavy chain

SEQ ID NO: 13

gcggccgccatgaattttggactgaggctgattttcctggtgctgaccctgaaaggcgtccagt
gtcaagtccagctcgtccagagcggggcggaagtcaagaagccgggggcgagcgtgaaagtctcgtgcaa
ggcgagcggatatacgttcacgaactactggattcactgggtccggcaagcgccggggcaggggctggag
tggatcgggaacatcgacccgtcggacacgcggacgcactacaaccagaagttcaaggaccgggcgacgc
tgaccgtcgacaagagcacgagcacggcgtacatggagctgtcgagcctgcggagcgaggacacggccgt
ctactactgcgcgcggcagacgctgtactacgaggcgctggactactggggcaggggacgctcgtcacg
gtctcgagcgctagcacaaagggccctagtgtgtttcctctggctccctcttccaaatccacttctggtg
gcactgctgctctgggatgcctggtgaaggattactttcctgaacctgtgactgtctcatggaactctgg
tgctctgacttctggtgtccacactttccctgctgtgctgcagtctagtggactgtactctctgtcatct
gtggtcactgtgccctcttcatctctgggaacccagacctacatttgtaatgtgaaccacaaaccatcca
acactaaagtggacaaaagagtggaacccaaatcctgtgacaaaacccacacctgcccaccttgtccggc

gcctgaagcggaaggaggaccttctgtgtttctgttccccccaaaccaaaggataccctgatgatctcg
cgaacccctgaggtgacatgtgtggtggtggatgtgtctcatgaggaccccgaagtcaaatttaattggt
atgtcgacggcgtcgaggtgcataatgccaaaaccaagcctagagaggaacagtacaattcaacctacag
agtcgtcagtgtgctgactgtgctgcatcaggattggctgaatggcaaggaatacaagtgtaaagtctca
aacaaggccctgcctgcttcaattgagaaaacaatctcaaaggccaagggacagcctagggaaccccagg
tctacaccctgccaccttcaagagaggaaatgaccaaaaaccaggtgtccctgacatgcctggtcaaagg
cttctacccttctgacattgctgtggagtgggagtcaaatggacagcctgagaacaactacaaaacaacc
ccccctgtgctggattctgatggctctttctttctgtactccaaactgactgtggacaagtctagatggc
agcaggggaatgtcttttcttgctctgtcatgcatgaggctctgcataaccactacactcagaaatccct
gtctctgtctcccgggaaatgataagctttgatgtcatgcatgaggctctgcataaccactacactcaga
aatccctgtctctgtctcccgggaaatgataagctttga

### Light chain

same as 2B (see SEQ ID NO: 10)
   > Heavy chain (SEQ ID NO: 7) 
QVQLVQSGAEVKKPGASVKVSCKASGYTFTNYWIHWVRQAPGQGLEWIGNIDPSDTRTH
YNQKFKDRATLTVDKSTSTAYMELSSLRSEDTAVYYCARQTLYYEALDYWGQGTLVTVSSASTK
GPSVFPLAPSSKSTSGGTAALGCLVKDYFPEPVTVS
WNSGALTSGVHTFPAVLQSSGLYSLSSVVTVPSSSLGTQTYICNVNHKPSNTKVDKRVE
PKSCDKTHTCPPCPAPEAEGGPSVFLFPPKPKDTLMISRTPEVTCVVVDVSHEDPEVKFNWYVD
GVEVHNAKTKPREEQYNSTYRVVSVLTVLHQDWLNG
KEYKCKVSNKALPASIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQK
SLSLSPGK
 > Light chain
same as 2B (see SEQ ID NO: 4)

### 7.2- SPR affinity determination of #2B1 and #2B2 humanized antibodies

The affinity constants (KD) of the pro-sPLA2-GIB and sPLA2-GIB binding to #2B1 and #2B2 were determined by surface kinetics on a real-time biosensor surface plasmon resonance assay (BIAcore™). The antibody was captured on an anti-mouse IgG surface and exposed to various concentrations of recombinant pro-sPLA2-IB and sPLA2-IB protein. Kinetic analysis using BIAevaluation™ software was performed to obtain the association and dissociation rate constants (Table 7). As presented in Table 7, the introduction of the double or the triple mutations in #2B1 or #2B2 did not impact significantly the affinity of the variants for the sPLA2-IB target.

### 7.3 - Differential Scanning Calorimetry of #2B1 and #2B2 humanized antibodies

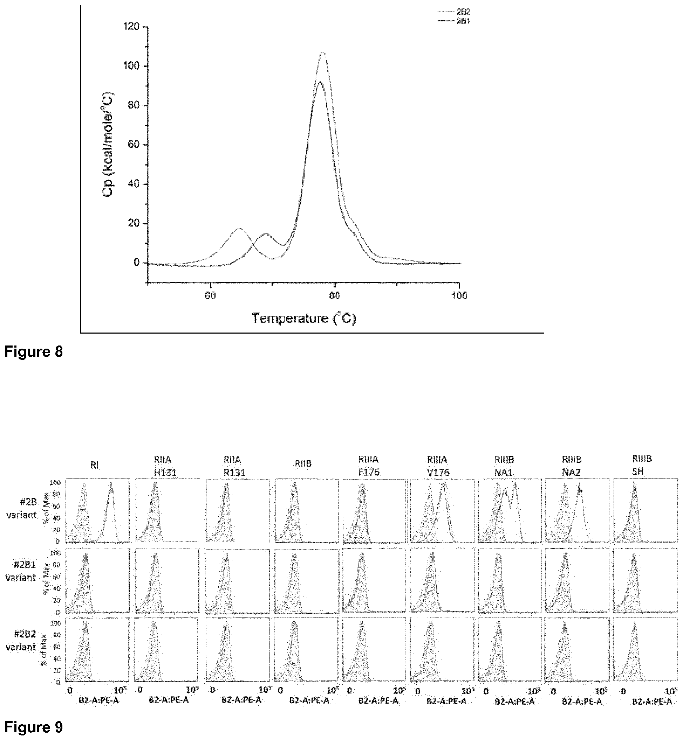

Normalized thermal denaturation of #2B1 and #2B2 are presented in Figure 8 and DSC parameters in Table 8. #2B1 shows a better profile in these experiments as the Tm onset and the Tm1 (corresponding to CH2) were higher.

### 7.4- Analytical Size Exclusion Chromatography (SEC-HPLC) of #2B1 and #2B2 humanized antibodies

#2B1 and #2B2 were analyzed by SEC-HPLC in PBS buffer. Results are summarized in Table 9. As presented, less than 5% of the mAb are present in high molecular weight aggregates, indicating monomeric conformation and absence of aggregation in the tested conditions.

### 7.5 - Capillary isoelectric focusing analysis of #2B1 and #2B2 humanized antibodies

The iso-electric point and relative distribution of charge variants of the #2B, #2B1 and #2B2 were determined by imaged capillary isoelectric focusing (icIEF) analysis. Results are summarized in Table 10. For all antibodies, repeatable results were obtained with low % RSD values. The pI of the main peak was 8.96 for #2B, 8.76 for #2B1 and 8.75 for #2B2. Besides a slightly different pI of the main peak, #2B also shows a slightly different charge variant profile as opposed to the other two samples. While #2B1 and #2B2 have more basic than acidic charge, the opposite is true for #2B (Table 10). The main peak in #2B comprised 64% of all peaks while in #2B1 and #2B2 the main peak only comprised 57%. No pre-acidic and post-basic peaks were detected in all tested samples.

### 7.6 -Binding of #2B, #2B1 and #2B2 to human FcγRs

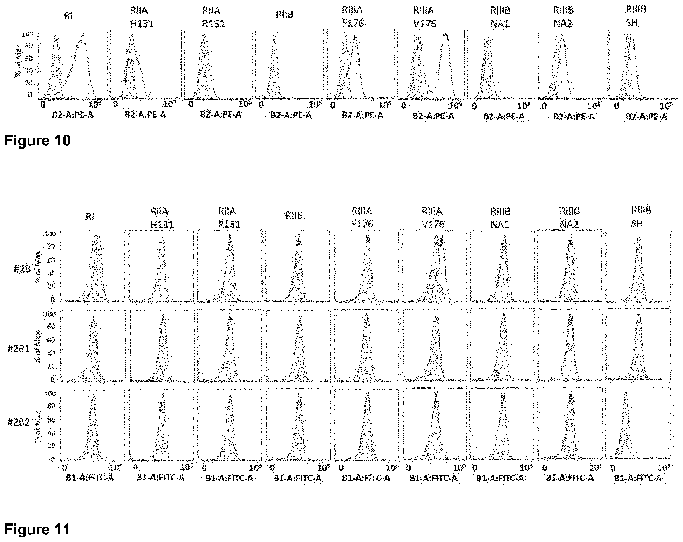

The binding of monomeric #2B, #2B1 and #2B2 to human FcγRs was tested by flow cytometry by incubating the mAbs with CHO cells stably expressing human FcγRI, FcγRIIA (H131), FcyRIIA(R131), FcγRIIB, FcyRIIIA(F176), FcγRIIIA(V176), FcγRIIIB(NA1), FcγRIIIB(NA2), and FcγRIIIB(SH), (*REF P. Bruhns*). As presented in Figure 9, #2B is binding to hFcγRI and hFcγRIIIA(V176) only, whereas no monomeric binding of #2B1 and #2B2 to all tested hFcγRs was observed. Antibody-antigen immune complex was tested by first incubating the mAb with the antigen and then, incubating the immune complexes formed with CHO cells stably expressing human FcγRs. The immune complexe of #2B is binding to all tested hFcγRs, except hFcyRIIB (Figure 10). Weak binding is observed for immune complex of #2B1 to hFcγRIIIA(V176) (data not shown), whereas no immune complex binding of #2B2 to any tested hFcγRs (data not shown). The residual binding of antigen on antibody-sensitized cells was tested by firstly incubating #2B, #2B1 and #2B2 with CHO cells stably expressing human FcγRs. After washing, the sPLA2-GIB biotinylated antigen was added and detected by flow cytometry using an avidin-FITC conjugate. As depicted in Figure 11, residual binding of the sPLA2-GIB antigen onto mAb #2B was observed when bound to hFcγRI and hFcγRIIIA(V176). In contrast, no residual binding of the sPLA2-GIB antigen onto mAb #2B1 and #2B2 was observed.

### 7.7 - Co-crystallization of #2B2 Fab with sPLA2-GIB and definition of the epitope

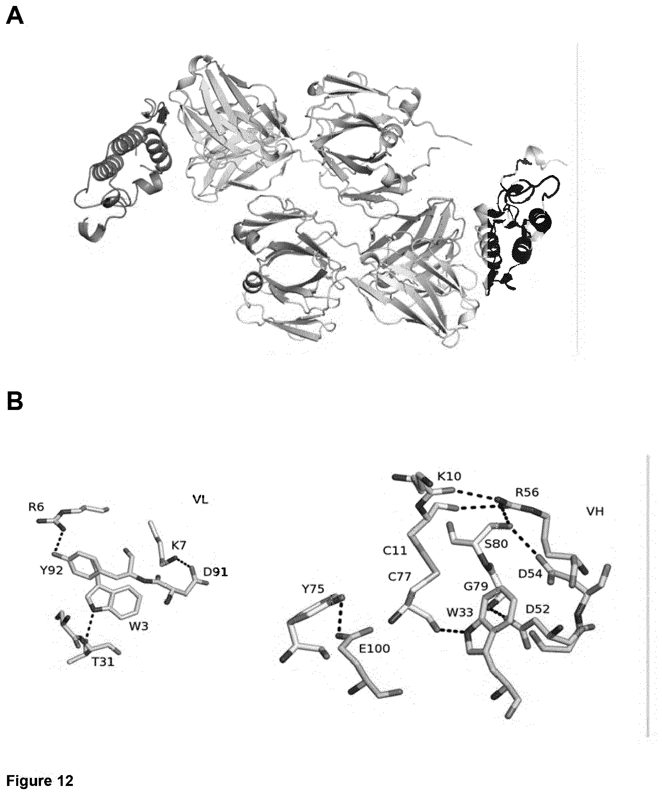

The Fab fragment of #2B2 was co-crystallized with its antigen, sPLA2-GIB and x-ray diffraction data of the co-crystal were collected on the PROXIMA-1 Beamline at the Soleil Synchrotron (St. Aubin, France). The crystals belong to the P21 space group (a=71.9Å, b=83.8Å, c=103.9Å; α=β=99.58°) with two sPLA2-GIB/Fab complex per asymmetric unit (Figure12A). The resolution of the structure of the sPLA2-GIB/Fab complex enabled the identification of the critical residues involved in the non-covalent interactions in the complex and definition of the #2B2 epitope. As presented in the Figure 12B, W3, R6, and K7 in the sPLA2-IB are involved in the interaction with the Fab light chain, and K10, C77, Y75, G79 and S80 in the sPLA2-GIB are involved in the interaction with the Fab heavy chain.

### 8- Biological properties of #2B2 humanized antibody

### 8.1- In vitro effect of #2B humanized antibody on the inhibition of phosphoSTAT5 nuclear translocation by sPLA2-GIB or plasma from HIV-infected patient.

The ability of #14G9 or #2B to counterbalance the inhibitory effect of 75 nM sPLA2-GIB or plasma isolated from HIV-1 infected patients on the IL-7 induced phospho-STAT5 nuclear translocation in CD4 T cells was tested by incubating plasmas isolated from HIV-1 infected patients with the #14G9 or the #2B mAbs.

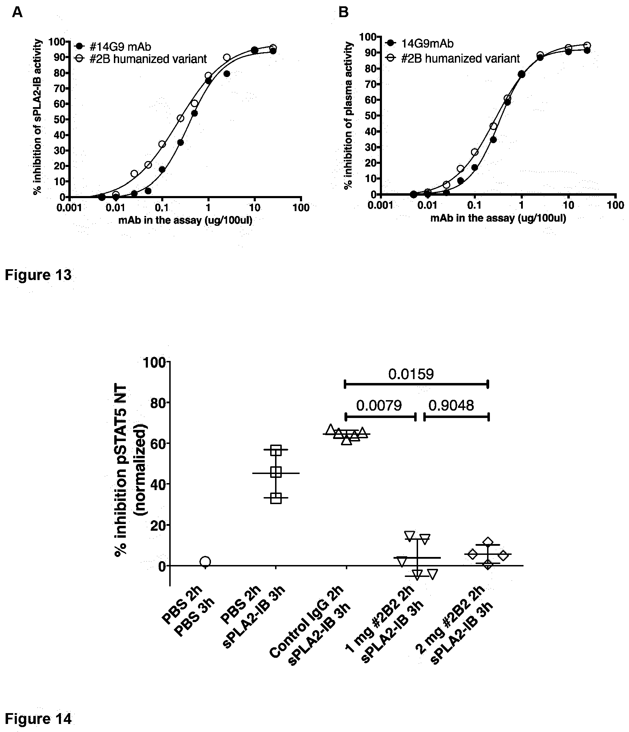

As depicted in Figure 13, increasing amounts of the #14G9 or #2B mAbs led to a dose dependent restoration of the ability of sPLA2-IB (Figure 13A) or plasmas from viremic patients (Figure 13B) to inhibit the nuclear translocation of phosphoSTAT5 induced by IL-7 in human CD4 T cells isolated from one healthy donor. EC50 for the restoration of the sPLA2-IB effect were 3.7 ng/mL and 2.2 ng/mL for #2B and the #14G9 mAb, respectively. EC50 for the restoration of the HIV infected plasma effect were 3.3 ng/mL and 2.7 ng/mL for the #2B and the #14G9 mAb, respectively.

### 8.2- In vivo effect of #2B2 on the inhibition of phosphoSTAT5 nuclear translocation by sPLA2-GIB

The ability of the #2B2 mAb to counterbalance *in vivo* the inhibitory effect of the injection of sPLA2-IB on the IL-7 induced phospho-STAT5 nuclear translocation in CD4 T cells was tested by first injecting 1 or 2 mg of the #2B2 mAb in mice, followed by the injection of 100 µg of sPLA2-IB. Nuclear translocation of phospho-STAT5 in CD4 T cells isolated from splenocytes of the treated mice was then analyzed by confocal microscopy.

As depicted in Figure 14, the injection of 100 µg of sPLA2-IB in mice led to a 50% inhibition of the nuclear translocation of phosphoSTAT5 in CD4 T cells isolated from splenocytes. This effect was fully abolished by the prior injection of 1 or 2 mg of the #2B2 mAb, with no significant effect observed in control injections.

**Table 1**

| IMGT Human germline | Variable regions | | | Number of identical residues |
|---|---|---|---|---|
| | Hybridoma | Humanized | | |
| | | version A | version B | |
| | | overall % identity | | |
| IGHV1-46*01 | 66.3 | 80.6 | 81.6 | 65 - 79-80 / 98 |
| IGHV5-10-1*04 | 58.2 | 80.6 | 81.6 | 57 - 79-80 / 98 |
| IGKV1-39*01 | 67.4 | 83.2 | 84.2 | 64 - 79-80 / 95 |
| IGKV3-15*01 | 62.1 | 85.3 | 86.3 | 59 - 81-82/ 95 |

**Table 2**

| Kabat nomenclature | WR1 | DR1 | C | F | DR2 | C | WR3 | F | DR3 | C | | | | |
|---|---|---|---|---|---|---|---|---|---|---|---|---|---|---|
| Humanized versions | Mismatching with human germ-line | | | | | | | | | | otal | - | | |
| **VH number of residues** | **0** | | **5** | **4** | **1** | **7** | **1** | **2** | **3** | | | **98** | **identit y** | |
| IGHV1 -46*01 version A | | | 3 | 2 | | 9 | | 5 | R | N | 9 | ' | 0.6% (79//98) | 8 |
| IGHV1 -46*01 version B | | | 3 | 1 | | 9 | | 5 | R | N | 8 | ' | 1.6% (80//98) | 8 |
| IGHV5 -10-1*04 version A | | | 2 | 2 | | 9 | | 4 | R | N | 9 | ' | 0.6% (79//98) | 8 |
| IGHV5 -10-1*04 version B | | | 2 | 1 | | 9 | | 4 | R | N | 8 | ' | 1.6% (80//98) | 8 |

| **VL number of residues** | **3** | | **6** | **1** | **5** | **1** | **7** | **2** | **3** | | | **100** | **identit y** | |
|---|---|---|---|---|---|---|---|---|---|---|---|---|---|---|
| IGKV1 -39*01 version A | | | 7 | | 1 | | 5 | 2 | | 1 | 6 | ' | 3.2% (79/95) | 8 |
| IGKV1 -39*01 version B | | | 7 | | 0 | | 5 | 2 | | 1 | 5 | ' | 4.2% (80/95) | 8 |
| IGKV3 -15*01 version A | | | 5 | | 1 | | 2 | 2 | | 4 | 4 | ' | 5.3% (81/95) | 8 |
| IGKV3 -15*01 version B | | | 5 | | 0 | | 2 | 2 | | 4 | 3 | ' | 6.3% (82/95) | 8 |

**Table 3**

| | Inhibition of Enzymatic Activity (% of Control) | | | |
|---|---|---|---|---|
| mAb (ug) | Ab#1A | Ab #1B | Ab #2A | Ab #2B |
| 0.1 | 64% | 60% | 55% | 42% |
| 0.3 | 41% | 42% | 33% | 24% |
| 1 | 19% | 22% | 20% | 13% |
| 3 | 14% | 12% | 12% | 10% |

**Table 4**

| Humanized Ab | pro-sPLA2-GIB IC50 (nM) | sPLA2-GIB IC50 (nM) |
|---|---|---|
| #1A | 1053 | 1.9 |
| #1B | 263 | 3.3 |
| #2A | 216 | 1.9 |
| #2B | 341 | 2.3 |

**Table 5**

| S ample name | C onc (mM) | T _{1/2} (°C) | Δ H (cal/M) | T ₒₙₛₑₜ (°C) | T ₘ₁ (°C) | T ₘ₂ (°C) | T ₘ₃ (°C) |
|---|---|---|---|---|---|---|---|
| A b #1A | 0. 0034 | 5 .01 | 1. 04E6 | 6 5.26 | 7 0.65 | 7 8.84 | - |
| A b #1B | 0. 0137 | 5 .01 | 1.09E6 | 6 4.28 | 7 1.19 | 8 1.94 | - |
| A b #2A | 0. 0078 | 5 .00 | 1. 06E6 | 6 4.76 | 7 4.15 | - | 8 2.70 |
| A b #2B | 0. 0074 | 5 .02 | 1. 03E6 | 6 4.68 | 7 0.59 | 7 7.81 | 8 3.85 |

**Table 6**

| | Retention Time | Area % | Calculated MW (kDa) |
|---|---|---|---|
| #1A Ab | 6.186 | 97.55 | 158 |
| #1B Ab | 6.193 | 97.19 | 157 |
| #2A Ab | 6.181 | 93.96 | 159 |
| #2B Ab | 6.183 | 97.52 | 159 |

**Table 7**

| mAb | Antigen | kon (10⁴ M⁻¹.s⁻¹) | koff (10⁻⁴ s⁻¹) | t1/2 (min) | Kd (pM) |
|---|---|---|---|---|---|
| #2B | sPLA2- IB | 289 ± 12 | 22.5 ± 2.0 | 5.1 ± 0.4 | 786 ± 63 |
| #2B1 | sPLA2- IB | 278 ± 1 | 20.3 ± 0.4 | 5.7 ± 0.1 | 729 ± 9 |
| #2B2 | sPLA2- IB | 302 ± 2 | 24.7 ± 1.3 | 4.7 ± 0.2 | 796 ± 8 |
| #2B | pro- sPLA2-IB | 17.6 ± 0.4 | 1417 ± 25 | 0.080 ± 0.002 | 784900 ± 31600 |
| #2B1 | pro-sPLA2-IB | 18.1 ± 0.4 | 1551 ± 51 | 0.074 ± 0.003 | 853400 ± 12400 |
| #2B2 | pro-sPLA2-IB | 16.4 ± 1.2 | 1462 ± 129 | 0.079 ± 0.007 | 879500 ± 28300 |

**Table 8**

| ample name S | C onc (mM) | T 1/2 (°C) | Δ H (cal/M) | T onset (°C) | T m1 (°C) | T m2 (°C) | T m3 (°C) |
|---|---|---|---|---|---|---|---|
| b #2B1 A | 0. 0060 | 5 .02 | 0. 591E6 | 6 4.16 | 6 8.66 | 7 7.60 | 8 3.38 |
| b #2B2 A | 0. 0074 | 5 .01 | 0.785E6 | 5 6.97 | 6 4.53 | 7 8.00 | 8 4.10 |

**Table 9**

| | Retention Time | Area % | Calculated MW (kDa) |
|---|---|---|---|
| Ab #2B1 | 6.128 | 96.30 | 170.53 |
| Ab #2B2 | 6.159 | 97.18 | 166.12 |

**Table 10**

| Sa mple name | A cidic 3 | A cidic 2 | A cidic 1 | M ain Peak | B asic 1 | B asic 2 | B asic 3 |
|---|---|---|---|---|---|---|---|
| Ab #2B | 8 .76 | 8 .84 | 8 .89 | 8 .96 | 9 .06 | 9 .13 | N A |
| Ab #2B1 | 8 .36 | 8 .50 | 8 .64 | 8 .76 | 8 .88 | 8 .99 | 9 .05 |
| Ab #2B2 | 8 .35 | 8 .49 | 8 .63 | 8 .75 | 8 .87 | 8 .98 | 9 .04 |

### REFERENCES

Eskola, J.U., Nevalainen, T.J. & Aho, H.J., 1983a. Purification and Characterization of Human Pancreatic Phospholipase A2. , 29(10), pp.1772-1776.
Eskola, J.U., Nevalainen, T.J. & Aho, H.J., 1983b. Purificationand Characterization of Human Pancreatic Phospholipase A2. , 29(10), pp.1772-1776.
Kitagawa, M. et al., 1991. The diagnostic value of serum pancreatic phospholipase A2 (PLA2) in pancreatic diseases. Gastroenterologia Japonica, 26(1), pp.62-68.
Kolko, M. et al., 2007. Human secretory phospholipase A2, group IB in normal eyes and in eye diseases. Acta Ophthalmologica Scandinavica, 85(3), pp.317-323.
Lambeau, G. & Gelb, M.H., 2008. Biochemistry and physiology of mammalian secreted phospholipases A2. Annual review of biochemistry, 77, pp.495-520. Available at: http://www.ncbi.nlm.nih.gov/pubmed/18405237.
Nakano, T. et al., 1994. Plasmin converts pro-form of group I phospholipase A2 into receptor binding, active forms. Biochemical and biophysical research communications, 198(1), pp. 10-15.
Nevalainen, T.J. et al., 1985. Immunoreactive phospholipase A2 in serum in acute pancreatitis and pancreatic cancer. Clin Chem, 31(7), pp.1116-1120. Available at: http://www.ncbi.nlm.nih.gov/pubmed/2408788.
Nevalainen, T.J. & Haapanen, T.J., 1993. Distribution of pancreatic (group I) and synovial-type (group II) phospholipases A2 in human tissues. Inflammation, 17(4), pp.453-464.
Nishijima, J. et al., 1983. Purification and characterization of human pancreatic phospholipase A2 and development of a radioimmunoassay. Journal of biochemistry, 94(1), pp. 137-147.
Ramanadham, S. et al., 1998. Type IB secretory phospholipase A2 is contained in insulin secretory granules of pancreatic islet ??-cells and is co-secreted with insulin from glucose-stimulated islets. Biochimica et Biophysica Acta - Lipids and Lipid Metabolism, 1390(3), pp.301-312.
Rouault, M. et al., 2007. Recombinant production and properties of binding of the full set of mouse secreted phospholipases A2 to the mouse M-type receptor. Biochemistry, 46(6), pp.1647-1662.
Singer, A.G. et al., 2002. Interfacial kinetic and binding properties of the complete set of human and mouse groups I, II, V, X, and XII secreted phospholipases A2. Journal of Biological Chemistry, 277(50), pp.48535-48549.
Sternby, B., 1984. IMMUNOREACTIVE PANCREATIC COLIPASE, LIPASE AND P H O SPHOLIPASEAz IN HUMAN PLASMA AND URINE FROM HEALTHY INDIVIDUALS. , 789, pp.164-169.
THEZE Jacques, Thierry, R. & BUGAULT Florence, 2015. Patent application WO2015097140A1.pdf.

## Claims

1. An isolated human or humanized antibody, or a derivative thereof, that binds human sPLA2-GIB.

2. The antibody of claim 1, or the derivative thereof, which is monoclonal.

3. The antibody or derivative of claim 1 or 2, which binds an epitope comprising at least one amino acid residue selected from W3, R6, K7, K10, C77, Y75, G79 and S80 of human sPLA2-GIB, by reference to SEQ ID NO: 14.

4. The antibody or derivative of claim 1 or 2, which competitively inhibits the binding of monoclonal antibody 14G9 to human sPLA2-GIB, said monoclonal antibody 14G9 comprising a light chain variable region comprising SED ID NO: 2 and a heavy chain variable region comprising SEQ ID NO: 3.

5. The antibody or derivative of claim 1 or 2, which competitively inhibits the binding of monoclonal antibody #2B, to human sPLA2-GIB, said monoclonal antibody #2B comprising a light chain comprising or consisting of SEQ ID NO: 4 and a heavy chain comprising or consisting of SEQ ID NO: 5.

6. The antibody or derivative of claim 1, which competitively inhibits the binding of monoclonal antibody #2B1, to human sPLA2-GIB, said monoclonal antibody #2B1 comprising a light chain comprising or consisting of SEQ ID NO: 4 and a heavy chain comprising or consisting of SEQ ID NO: 6.

7. The antibody or derivative of claim 1, which competitively inhibits the binding of monoclonal antibody #2B2, to human sPLA2-GIB, said monoclonal antibody #2B2 comprising a light chain comprising or consisting of SEQ ID NO: 4 and a heavy chain comprising or consisting of SEQ ID NO: 7.

8. The antibody or derivative of anyone of the preceding claims, which neutralizes sPLA2-GIB.

9. The isolated antibody of claim 1, which comprises a light chain comprising or consisting of SEQ ID NO: 4 and a heavy chain comprising or consisting of SEQ ID NO: 5.

10. The isolated antibody of claim 1, which comprises a light chain comprising or consisting of SEQ ID NO: 4 and a heavy chain comprising or consisting of SEQ ID NO: 6.

11. The isolated antibody of claim 1, which comprises a light chain comprising or consisting of SEQ ID NO: 4 and a heavy chain comprising or consisting of SEQ ID NO: 7.

12. A nucleic acid encoding an antibody or derivative of anyone of the preceding claims, or a light or heavy chain of such an antibody, or a variable domain thereof.

13. A nucleic acid of claim 12, comprising a nucleotide sequence selected from anyone of SEQ ID NOs: 10-13 or the complement thereof.

14. A vector comprising a nucleic acid of claim 12 or 13.

15. A recombinant host cell containing a vector of claim 14 or a nucleic acid of claim 13.

16. A pharmaceutical composition comprising (i) an antibody or derivative of any one of claims 1 to 11, or a vector of claim 14 or a nucleic acid of claim 12 or a recombinant host cell of claim 15, and (ii) a pharmaceutically acceptable carrier or excipient.

17. An antibody or derivative of any one of claims 1 to 11 for use in treatment of a disease in a subject in need thereof.

18. A method for producing an antibody of any of claims 1 to 11, comprising culturing the cell of claim 15 under conditions suitable for expression of the antibody, and optionally recovering said antibody.


SEQUENCE LISTING

<110>  DIACCURATE

<120>  THERAPEUTIC ANTI-sPLA2-GIB ANTIBODIES

<130>  B2701

<160>  14

<170>  PatentIn version 3.5

<210>  1
<211>  148
<212>  PRT
<213>  Homo sapiens

<400>  1

Met Lys Leu Leu Val Leu Ala Val Leu Leu Thr Val Ala Ala Ala Asp
1               5                   10                  15

Ser Gly Ile Ser Pro Arg Ala Val Trp Gln Phe Arg Lys Met Ile Lys
            20                  25                  30

Cys Val Ile Pro Gly Ser Asp Pro Phe Leu Glu Tyr Asn Asn Tyr Gly
        35                  40                  45

Cys Tyr Cys Gly Leu Gly Gly Ser Gly Thr Pro Val Asp Glu Leu Asp
    50                  55                  60

Lys Cys Cys Gln Thr His Asp Asn Cys Tyr Asp Gln Ala Lys Lys Leu
65                  70                  75                  80

Asp Ser Cys Lys Phe Leu Leu Asp Asn Pro Tyr Thr His Thr Tyr Ser
                85                  90                  95

Tyr Ser Cys Ser Gly Ser Ala Ile Thr Cys Ser Ser Lys Asn Lys Glu
            100                 105                 110

Cys Glu Ala Phe Ile Cys Asn Cys Asp Arg Asn Ala Ala Ile Cys Phe
        115                 120                 125

Ser Lys Ala Pro Tyr Asn Lys Ala His Lys Asn Leu Asp Thr Lys Lys
    130                 135                 140

Tyr Cys Gln Ser
145


<210>  2
<211>  117
<212>  PRT
<213>  Artificial Sequence

<220>
<223>  14G9 VL

<400>  2

Asp Ile Val Met Thr Gln Ser His Lys Leu Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Glu Lys Pro Gly Gln Ser Pro Lys Leu Leu Ile
        35                  40                  45

Tyr Trp Ala Ser Thr Arg His Thr Gly Val Pro Asn Arg Phe Thr Gly
    50                  55                  60

Ser Arg Ser Gly Thr Asp Tyr Thr Leu Thr Ile Ser Ser Val Gln Ala
65                  70                  75                  80

Glu Asp Leu Ala Leu Tyr Tyr Cys Gln Gln Asp Tyr Ser Thr Pro Pro
                85                  90                  95

Thr Phe Gly Ala Gly Thr Lys Leu Glu Leu Lys Arg Ala Asp Ala Ala
            100                 105                 110

Pro Thr Val Ser Ile
        115


<210>  3
<211>  130
<212>  PRT
<213>  Artificial Sequence

<220>
<223>  14G9 VH

<400>  3

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Ile His Trp Val Lys Arg Arg Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Lys Ala Thr Leu Ala Val Asp Lys Ser Ser Ser Thr Ala Tyr
65                  70                  75                  80

Met Gln Leu Ser Ser Leu Thr Ser Glu Asp Ser Ala Val Phe Tyr Cys
                85                  90                  95

Ala Arg Gln Thr Leu Tyr Tyr Glu Ala Leu Asp Tyr Trp Gly Gln Gly
            100                 105                 110

Thr Thr Leu Thr Val Ser Ser Ala Lys Thr Thr Pro Pro Ser Val Tyr
        115                 120                 125

Pro Leu
    130

<210>  4
<211>  214
<212>  PRT
<213>  Artificial Sequence

<220>
<223>  2B L

<400>  4

Glu Ile Val Met Thr Gln Ser Pro Ala Thr Leu Ser Val Ser Pro Gly
1               5                   10                  15

Glu Arg Ala Thr Leu Ser Cys Lys Ala Ser Gln Asp Val Ser Thr Ala
            20                  25                  30

Val Ala Trp Tyr Gln Gln Lys Pro Gly Gln Ala Pro Arg Leu Leu Ile
        35                  40                  45

Tyr Trp Ala Ser Thr Arg His Thr Gly Ile Pro Ala Arg Phe Ser Gly
    50                  55                  60

Ser Arg Ser Gly Thr Glu Tyr Thr Leu Thr Ile Ser Ser Leu Gln Ser
65                  70                  75                  80

Glu Asp Phe Ala Val Tyr Tyr Cys Gln Gln Asp Tyr Ser Thr Pro Pro
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  5
<211>  449
<212>  PRT
<213>  Artificial Sequence

<220>
<223>  2B H

<400>  5

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Trp Ile His Trp Val Arg Gln Ala Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Leu Tyr Tyr Glu Ala Leu Asp Tyr Trp Gly Gln Gly
            100                 105                 110

Thr Leu Val Thr Val Ser Ser Ala Ser Thr Lys Gly Pro Ser Val Phe

115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  6
<211>  449
<212>  PRT
<213>  Artificial Sequence

<220>
<223>  2B1 H

<400>  6

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Trp Ile His Trp Val Arg Gln Ala Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Leu Tyr Tyr Glu Ala Leu Asp Tyr Trp Gly Gln Gly
            100                 105                 110

Thr Leu Val Thr Val Ser Ser Ala Ser Thr Lys Gly Pro Ser Val Phe

115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Ala Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  7
<211>  449
<212>  PRT
<213>  Artificial Sequence

<220>
<223>  2B2 H

<400>  7

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Trp Ile His Trp Val Arg Gln Ala Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Leu Tyr Tyr Glu Ala Leu Asp Tyr Trp Gly Gln Gly
            100                 105                 110

Thr Leu Val Thr Val Ser Ser Ala Ser Thr Lys Gly Pro Ser Val Phe

115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Ala Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  8
<211>  351
<212>  DNA
<213>  Artificial Sequence

<220>
<223>  14G9 VL

<400>  8
gacattgtga tgacccagtc tcacaaactc atgtccacat cagtaggaga cagggtcagc      60

atcacctgca aggccagtca ggatgtgagt actgctgtag cctggtatca agaaaaacca     120

ggtcaatctc ctaaactact gatttactgg gcatccaccc ggcacactgg agtccctaat     180

cgcttcacag gcagtagatc tgggacagat tatactctca ccatcagcag tgtgcaggct     240

gaagacctgg cactttatta ctgtcagcaa gattatagca ctcctcctac gttcggtgct     300

gggaccaagc tggagctgaa acgggctgat gctgcaccaa ctgtatccat c              351

<210>  9
<211>  392
<212>  DNA
<213>  Artificial Sequence

<220>
<223>  14G9 VH

<400>  9
caggtccaac tgcaacagcc tggggctgag ctggtgaagc ctggggcttc agtgaagctg      60

tcctgcaagg cttctggcta caccttcacc aactactgga tacactgggt gaagcggagg     120

cctggacaag gccttgaatg gattggtaat attgaccctt ctgatactag aactcactac     180

aatcaaaagt tcaaggacaa ggccacattg gctgtagaca aatcctccag cacagcctac     240

atgcagctca gcagcctgac atctgaggac tctgcggtct tttactgtgc aagacagacc 300

ctctactatg aggccttgga ctactggggc caaggcacca ctctcacagt ctcctcagcc 360

aaaacgacac ccccatctgt ctatccactg gg 392

<210> 10
<211> 726
<212> DNA
<213> Artificial Sequence

<220>
<223> 2B L

<400> 10
gcggccgcca tgaattttgg actgaggctg attttcctgg tgctgaccct gaaaggcgtc 60

cagtgtgaga tcgtgatgac gcagtcgccg gcgacgctga gcgtgagtcc gggggagcgg 120

gcgacgctga gctgcaaggc gtcgcaggac gtgagcacgg ccgtcgcgtg gtatcaacag 180

aagccggggc aagcgccgcg gctgctgatc tactgggcga gcacgcggca cacgggcatc 240

ccggcgcgat tctcggggag ccggagcggg acggagtaca cgctgacgat ctcgtcgctg 300

caaagcgagg acttcgccgt ctactactgc cagcaggact actcgacgcc gccgacgttc 360

ggggggggta ccaaggtcga gatcaaacgt acggtcgcgg cgccttctgt gttcattttc 420

cccccatctg atgaacagct gaaatctggc actgcttctg tggtctgtct gctgaacaac 480

ttctacccta gagaggccaa agtccagtgg aaagtggaca atgctctgca gagtgggaat 540

tcccaggaat ctgtcactga gcaggactct aaggatagca catactccct gtcctctact 600

ctgacactga gcaaggctga ttacgagaaa cacaaagtgt acgcctgtga agtcacacat 660

caggggctgt ctagtcctgt gaccaaatcc ttcaataggg gagagtgctg atagtaaaag 720

ctttga 726

<210> 11
<211> 1431
<212> DNA
<213> Artificial Sequence

<220>
<223> 2B H

<400> 11
gcggccgcca tgaattttgg actgaggctg attttcctgg tgctgaccct gaaaggcgtc 60

cagtgtcaag tccagctcgt ccagagcggg gcggaagtca agaagccggg ggcgagcgtg 120

aaagtctcgt gcaaggcgag cggatatacg ttcacgaact actggattca ctgggtccgg 180

caagcgccgg ggcaggggct ggagtggatc gggaacatcg acccgtcgga cacgcggacg 240

cactacaacc agaagttcaa ggaccgggcg acgctgaccg tcgacaagag cacgagcacg 300

gcgtacatgg agctgtcgag cctgcggagc gaggacacgg ccgtctacta ctgcgcgcgg 360

cagacgctgt actacgaggc gctggactac tgggggcagg ggacgctcgt cacggtctcg 420

agcgctagca caaagggccc tagtgtgttt cctctggctc cctcttccaa atccacttct 480

ggtggcactg ctgctctggg atgcctggtg aaggattact ttcctgaacc tgtgactgtc 540

tcatggaact ctggtgctct gacttctggt gtccacactt tccctgctgt gctgcagtct 600

agtggactgt actctctgtc atctgtggtc actgtgccct cttcatctct gggaacccag 660

acctacattt gtaatgtgaa ccacaaacca tccaacacta aagtggacaa aagagtggaa 720

cccaaatcct gtgacaaaac ccacacctgc ccaccttgtc ctgcccctga actgctggga 780

ggaccttctg tgtttctgtt cccccccaaa ccaaaggata ccctgatgat ctctagaacc 840

cctgaggtga catgtgtggt ggtggatgtg tctcatgagg accctgaggt caaattcaac 900

tggtacgtgg atggagtgga agtccacaat gccaaaacca agcctagaga ggaacagtac 960

aattcaacct acagagtggt cagtgtgctg actgtgctgc atcaggattg gctgaatggc 1020

aaggaataca agtgtaaagt ctcaaacaag gccctgcctg ctccaattga gaaaacaatc 1080

tcaaaggcca agggacagcc tagggaaccc caggtctaca ccctgccacc ttcaagagag 1140

gaaatgacca aaaaccaggt gtccctgaca tgcctggtca aaggcttcta cccttctgac 1200

attgctgtgg agtgggagtc aaatggacag cctgagaaca actacaaaac aaccccccct 1260

gtgctggatt ctgatggctc tttctttctg tactccaaac tgactgtgga caagtctaga 1320

tggcagcagg ggaatgtctt ttcttgctct gtcatgcatg aggctctgca taaccactac 1380

actcagaaat ccctgtctct gtctcccggg aaatgatagt aaaagctttg a 1431

<210> 12
<211> 1426
<212> DNA
<213> Artificial Sequence

<220>
<223> 2B1 H

<400> 12
gcggccgcca tgaattttgg actgaggctg attttcctgg tgctgaccct gaaaggcgtc 60

cagtgtcaag tccagctcgt ccagagcggg gcggaagtca agaagccggg ggcgagcgtg 120

aaagtctcgt gcaaggcgag cggatatacg ttcacgaact actggattca ctgggtccgg 180

caagcgccgg ggcaggggct ggagtggatc gggaacatcg acccgtcgga cacgcggacg 240

cactacaacc agaagttcaa ggaccgggcg acgctgaccg tcgacaagag cacgagcacg 300

gcgtacatgg agctgtcgag cctgcggagc gaggacacgg ccgtctacta ctgcgcgcgg 360

cagacgctgt actacgaggc gctggactac tgggggcagg ggacgctcgt cacggtctcg 420

agcgctagca caaagggccc tagtgtgttt cctctggctc cctcttccaa atccacttct 480

ggtggcactg ctgctctggg atgcctggtg aaggattact ttcctgaacc tgtgactgtc 540

tcatggaact ctggtgctct gacttctggt gtccacactt tccctgctgt gctgcagtct 600

agtggactgt actctctgtc atctgtggtc actgtgccct cttcatctct gggaacccag 660

acctacattt gtaatgtgaa ccacaaacca tccaacacta aagtggacaa aagagtggaa 720

cccaaatcct gtgacaaaac ccacacctgc ccaccttgtc cggcgcctga agcggaagga 780

ggaccttctg tgtttctgtt cccccccaaa ccaaaggata ccctgatgat ctcgcgaacc 840

cctgaggtga catgtgtggt ggtggatgtg tctcatgagg accccgaagt caaatttaat 900

tggtatgtcg acggcgtcga ggtgcataat gccaaaacca agcctagaga ggaacagtac 960

aattcaacct acagagtcgt cagtgtgctg actgtgctgc atcaggattg gctgaatggc 1020

aaggaataca agtgtaaagt ctcaaacaag gccctgcctg ctccaattga gaaaacaatc 1080

tcaaaggcca agggacagcc tagggaaccc caggtctaca ccctgccacc ttcaagagag 1140

gaaatgacca aaaaccaggt gtccctgaca tgcctggtca aaggcttcta cccttctgac 1200

attgctgtgg agtgggagtc aaatggacag cctgagaaca actacaaaac aaccccccct 1260

gtgctggatt ctgatggctc tttctttctg tactccaaac tgactgtgga caagtctaga 1320

tggcagcagg ggaatgtctt ttcttgctct gtcatgcatg aggctctgca taaccactac 1380

actcagaaat ccctgtctct gtctcccggg aaatgataag ctttga 1426

<210> 13
<211> 1503
<212> DNA
<213> Artificial Sequence

<220>
<223> 2B2 H

<400> 13
gcggccgcca tgaattttgg actgaggctg attttcctgg tgctgaccct gaaaggcgtc 60

cagtgtcaag tccagctcgt ccagagcggg gcggaagtca agaagccggg ggcgagcgtg 120

aaagtctcgt gcaaggcgag cggatatacg ttcacgaact actggattca ctgggtccgg 180

caagcgccgg ggcaggggct ggagtggatc gggaacatcg acccgtcgga cacgcggacg 240

cactacaacc agaagttcaa ggaccgggcg acgctgaccg tcgacaagag cacgagcacg 300

gcgtacatgg agctgtcgag cctgcggagc gaggacacgg ccgtctacta ctgcgcgcgg 360

cagacgctgt actacgaggc gctggactac tgggggcagg ggacgctcgt cacggtctcg 420

agcgctagca caaagggccc tagtgtgttt cctctggctc cctcttccaa atccacttct 480

ggtggcactg ctgctctggg atgcctggtg aaggattact ttcctgaacc tgtgactgtc 540

tcatggaact ctggtgctct gacttctggt gtccacactt tccctgctgt gctgcagtct 600

agtggactgt actctctgtc atctgtggtc actgtgccct cttcatctct gggaacccag 660

acctacattt gtaatgtgaa ccacaaacca tccaacacta aagtggacaa aagagtggaa 720

cccaaatcct gtgacaaaac ccacacctgc ccaccttgtc cggcgcctga agcggaagga 780

ggaccttctg tgtttctgtt cccccccaaa ccaaaggata ccctgatgat ctcgcgaacc 840

cctgaggtga catgtgtggt ggtggatgtg tctcatgagg accccgaagt caaatttaat 900

tggtatgtcg acggcgtcga ggtgcataat gccaaaacca agcctagaga ggaacagtac 960

aattcaacct acagagtcgt cagtgtgctg actgtgctgc atcaggattg gctgaatggc 1020

aaggaataca agtgtaaagt ctcaaacaag gccctgcctg cttcaattga gaaaacaatc 1080

tcaaaggcca agggacagcc tagggaaccc caggtctaca ccctgccacc ttcaagagag 1140

gaaatgacca aaaaccaggt gtccctgaca tgcctggtca aaggcttcta cccttctgac 1200

attgctgtgg agtgggagtc aaatggacag cctgagaaca actacaaaac aaccccccct 1260

gtgctggatt ctgatggctc tttctttctg tactccaaac tgactgtgga caagtctaga 1320

tggcagcagg ggaatgtctt ttcttgctct gtcatgcatg aggctctgca taaccactac 1380

actcagaaat ccctgtctct gtctcccggg aaatgataag ctttgatgtc atgcatgagg 1440

ctctgcataa ccactacact cagaaatccc tgtctctgtc tcccgggaaa tgataagctt 1500

tga 1503

<210> 14
<211> 126
<212> PRT
<213> Homo sapiens

<400> 14

Ala Val Trp Gln Phe Arg Lys Met Ile Lys Cys Val Ile Pro Gly Ser
1               5                   10                  15

Asp Pro Phe Leu Glu Tyr Asn Asn Tyr Gly Cys Tyr Cys Gly Leu Gly
            20                  25                  30

Gly Ser Gly Thr Pro Val Asp Glu Leu Asp Lys Cys Cys Gln Thr His
        35                  40                  45

Asp Asn Cys Tyr Asp Gln Ala Lys Lys Leu Asp Ser Cys Lys Phe Leu
    50                  55                  60

Leu Asp Asn Pro Tyr Thr His Thr Tyr Ser Tyr Ser Cys Ser Gly Ser
65                  70                  75                  80

Ala Ile Thr Cys Ser Ser Lys Asn Lys Glu Cys Glu Ala Phe Ile Cys
                85                  90                  95

Asn Cys Asp Arg Asn Ala Ala Ile Cys Phe Ser Lys Ala Pro Tyr Asn
            100                 105                 110

Lys Ala His Lys Asn Leu Asp Thr Lys Lys Tyr Cys Gln Ser
        115                 120                 125